# EUROPEAN PATENT SPECIFICATION

(11) **EP 0 751 947 B1**
(45) Date of publication and mention of the grant of the patent: **12.08.1998**
(21) Application number: 95928857.2
(22) Date of filing: 20.03.1995
(51) Int. Cl.: C07H 15/04, A61K 31/70

(54) **GLUCOSIDIC DERIVATIVES OF N-ACYL ALKYLAMINES EXERTING NEUROPROTECTIVE, NEUROTROPHIC AND ANTI-INFLAMMATORY ACTION, USEFUL IN ACUTE AND CHRONIC DISORDERS OF THE CENTRAL NERVOUS SYSTEM CONNECTED WITH EXCITOTOXICITY**
GLUKOSID-DERIVATE VON N-ACYL-ALKYLAMINEN MIT NEUROPROTEKTIVER,NEUROTROPHISCHER UND ENTZÜNDUNGSHEMMENDER WIRKUNG FÜR DIE BEHANDLUNG VON ZENTRALNERVENSYSTEMERKRANKUNGEN
DERIVES GLUCOSIDES D'ALCOYLAMINES N-ACYLE EXERçANT UNE ACTION NEUROPROTECTRICE, NEUROTROPHIQUE ET ANTI-INFLAMMATOIRE, UTILES DANS DES TROUBLES AIGUS ET CHRONIQUES DU SYSTEME NERVEUX CENTRAL ASSOCIES A UNE EXCITOTOXICITE

(30) Priority: 21.03.1994 IT MI940524
(43) Date of publication of application: 08.01.1997
(73) Proprietor: LIFEGROUP S.p.A., I-35043 Monselice (Padova) (IT)
(72) Inventor: DELLA VALLE, Francesco, I-35122 Padova (IT); LEON, Alberta, I-35142 Padova (IT); MARCOLONGO, Gabriele, I-35020 Carrara S. Giorgio (PD) (IT); DELLA VALLE, Federica, I-35123 Padova (IT)
(74) Representative: Gervasi, Gemma, Dr.
(86) International application number: EP9501026
(87) International publication number: WO9525736

(56) References cited:
- WO-A-94/03469
- CARBOHYDRATE RESEARCH, vol. 46, no. 1, January 1976 AMSTERDAM NL, pages 133-137, H. M. FLOWERS 'Synthesis of some long-chain acylamidoalkyl glucosides.'
- WENNER-GREN CENT. INT. SYMP. SER. (RECEPT.-RECEPT. INTERACT.), vol. 48, 1987 pages 54-61, G. TOFFANO ET AL 'Gangliosides as modulators of neuronotrophic interactions.'

## Description

### FIELD OF THE INVENTION

The present invention relates to glucosidic derivatives of N-acyl alkylamines therapeutically active in the treatment of disorders of the central nervous system connected with toxicity induced by Excitatory Amino Acids.

### PRIOR ART DISCLOSURE

As known the Nervous System is a sophisticated interneuronal communication network conveying information from the outside to the inside, where it is integrated.

Owing to the system functional role and complexity, the disorders of the central nervous system, because of the frequent seriousness of symptoms, are of great clinical importance and social impact, especially due to their disabling nature and to the age of affected people, frequently young.

Although the sequence of neurodegenerative events following cerebral damage depends on the etiopathogenetic characteristics of the specific event, the common final outcome is neuronal death. Thus a significant and correct therapeutic approach, which is finding increasing favour in the field of both acute and chronic neurodegenerative conditions of the central nervous system (CNS), is neuroprotection, which term refers to a strategy of pharmacological intervention in the stream of events causing neuronal death. In this paradigm, drugs must be capable of inhibiting the cytotoxic events following the pathogenic noxae altering neuronal equilibria.

Furthermore, such pharmacological intervention shold be able to promote repair processes by facilitating adaptive responses to pathogenetic noxae, a phenomenon known as neuronal plasticity.

Towards the end of the '50s, evidence was provided that some Amino Acids can excite single neurons of the CNS; in particular L-glutamic and L-aspartic acids were identified to be Amino Acids playing in the CNS the role of neurotransmitters. A successive development and use of competitive and non-competitive antagonists of Excitatory Amino Acids (EAA) evidenced that their biological effect is mediated by several receptors.

Said receptors are generally denominated - with regard to the exogenous agonist - N-methyl-D-aspartic acid (NMDA), quisqualic acid and kainic acid receptors. In particular, glutamic acid exerts its action through the activation of specific receptors that are pharmacologically and functionally subdivided into three classes: the metabotropic receptors bound to G-proteins and activated by quisqualic acid and functionally associated with phosphatidyl inositol hydrolysis; the receptors associated with an ionic channel selective for sodium and activated by the glutamic acid analogue named a-amino-3-hydroxy-5-methyl-4-isoxazol-propionic acid (AMPA); N-methyl-D-aspartic acid (NMDA) sensitive receptors associated with a cationic channel permeable to both sodium and calcium. The NMDA receptor response can also be influenced by substances acting cn particular sites, the so called allosteric modulation sites, such as for example glycine, which, even at low concentrations, improves the response to NMDA but does not affect the response to kainic or quisqualic acids. Conversely, the effects of aspartic acid are especially mediated by NMDA receptor.

Under physiological conditions, Excitatory Amino Acids (EAA) mediate the excitatory synaptic transmission involved in different processes of neuronal plasticity, underlying for example behavioural and cognitive phenomena (learning and memory), and in motor functions.

However, it is known that the same EAA receptors mediating the physiological neuronal depolarization, may be also responsible for neuronal damage: a prolonged and massive stimulation of EAA receptors, both NMDA and non-NMDA types, may result in CNS neuronal death. The mechanism or mechanisms underlying said cytotoxicity are extremely complex and involve neuronal depolarization, synapsys swelling. alteration of ionic homeostasis, i.e. an increased calcium influx and a potential activation of second messengers.

Several evidences have pointed out a correlation, at least in part, with an NMDA receptor overstimulation in neurological damage following acute events, such as for example hypoxic-ischaemia, stroke, hypoglycaemia, epilepsy, perinatal anoxia, brain and spinal cord injuries, degenerative chronic conditions such as Hungtington's Chorea, Alzheimer's and Parkinson's diseases or other forms of dementia (J.W. Olney, "Excitotoxic Amino Acids and Neuropsychiatric Disorders", *Ann.Rev.Pharmacol.Toxicol.,* 30: 47-71, 1990) as well as neurological deficits connected with viral diseases, such as e.g. HIV-1 infection (AIDS), for which excitatory amino acids, in particular quinolinic acid, are described to play a direct etiogenic role (M.P. Heyes et al.. "Quinolinic Acid in Cerebrospinal Fluid and Serum in HIV-1 Infection: Relationship to Clinical and Neurological Status", *Ann.Neurol.,* 29: 202-209, 1991). It is also worth mentioning, among these pathological conditions, neurolathyrism, amyotrophic lateral sclerosis, and pontocerebellar degeneration (J.W. Olney, op.cit., 1990). More recent researches also evidenced that L-glutamic acid is the primary excitatory transmitter on hypothalamic neurons level and, therefore, point out a primary role of said neurotransmitter in hypothalamic homeostasis and in the processes depending thereon (A.N. Van den Pol and P.Q. Trombley, *The J. of Neuroscience,* 13(7): 2829-2836, 1993).

It follows that the patho-physiological mechanisms at the basis of said diseases might be positively influenced by rational therapies capable of intervening in excitotoxic processes mediated by glutamatergic receptors. On the other side, neuronal plasticity and consequently neuronal repair are critically regulated by neurotrophic agents. Along this line, a correct therapeutical approach should be also directed to facilitate proneurotrophic effect.

In this context, the following therapeutic approaches were developed: i) competitive and non-competitive NMDA receptor antagonists, such as MK-801 which had initially been thought of as an anticonvulsant and later found to be capable of preventing ischaemic damage if administered in vivo at the time of damage induction: ii) calcium-antagonists, due to their effect on calcium channels blockage and, therefore, on the decrease in calcium influx into the cells; however, it has to be noted that this class of molecules does not exert any direct action on the excitotoxicity from L-glutamic acid; iii) neurotrophic agents, such as gangliosides, in particular monosialoganglioside GM1 and some single-chain semisynthetic derivatives thereof containing N-acetyl sphingosine, being capable of limiting the anoxic-ischaemic damage and, in vitro, the damage of EAA on primary neuronal cultures, under pretreatment and/or cotreatment conditions with respect to the exposure to the excitotoxic stimulus represented by L-glutamic acid.

Among the proposed therapies, the use of calcium antagonists is certainly limitative with respect to the generic and aspecific effect exerted. Conversely, the use of NMDA receptor antagonists may cause psychogenic side effects together with an impairment of the physiologically important plastic functions connected with cognitive and mnesic processes - which factors have so far hindered their clinical development and use. Different is the case of gangliosides, which have aroused a considerable interest in clinical practice because of their outstanding and amply described pharmacological activity modulating said neurodegenerative processes (A. Carolei et al., "Monosialoganglioside in Cerebral Ischemia", *Cerebrovascular and Brain metabolism Reviews,* 3, 134-157, 1991).

One of the neuroprotective mechanisms described is connected with the ability of GM1 and derivatives thereof of acting on protein kinase C (PKC) translocation and on consequent calcium accumulation with induction of the stream of biochemical events associated with, such as the activation of calcium-dependent enzymes, e.g. lipase and protease, events which might be the final effectors of neuronal death. In fact, a considerable increase in PKC translocation following an excessive exposure of neuronal cultures to L-glutamic acid was proved to be associated with a prolonged and excessive increase in cytosolic Ca⁺², which persists even after removal of L-glutamic acid or application of glutamic receptor blockers (H. Manev et al., "Glutamate induced Neuronal Death in Primary Cultures of Cerebellar Granule Cells: Protection by Synthetic Derivatives of Endogenous Sphingolipids", *J.PET,* 252 (1): 419-427, 1990).

It is also known that sphingosine and derivatives thereof are potent and reversible PKC inhibitors (J.A. Hannun and R.M. Bell, "Lysosphingolipids inhibit Protein Kinase C: Implication for the Sphingolipidase", *Science,* 235: 670-674, 1987). Therefore, the ability of inhibiting PKC activation is common to gangliosides (GM1, GT1b, GD1a, and GD1b), to GM1 derivatives and sphingosine. However, some aspects connected with sphingosine cytotoxicity, which are absent in gangliosides, but seem to be present, though to a limited extent, in some derivatives thereof, are to be furtner studied in said gangliosides lysoderivatives (H. Manev et al., op.cit., 1990).

It is still to be clarified whether the biological effect characteristic of said molecules is induced by intact, molecules per se, or by the intracellular production of active metabolites. In fact, experimental evidences concerning the ability of GM1 lysoderivatives to limit neuronal damage, even when orally administed (J.S. Schneider and L. Di Stefano, "Liga 20 increases striatal dopamine levels in aged MPTP-treated mice refractory to GM1 ganglioside treatment", *NeuroReport,* 5: 103-104, 1993) suggest that gangliosides/lysoderivatives metabolites may be pharmacologically active and that sphingosine and structural analogues thereof play a role in said activity.

### SUMMARY OF THE INVENTION

It has surprisingly been found that new glycosidic derivatives of N-acyl alkylamines obtained by synthesis are capable of exerting a protective action against the cytotoxicity induced by excitatory amino acids even when added before (pre-treatment), during (co-treatment) and more importantly after exposure to L-glutamic acid (post-treatment), in the absence of a significant intrinsic toxicity.

Therefore, the present invention is related to mono, di, and trisaccharide derivatives of N-acyl alkylamines of formula (I): 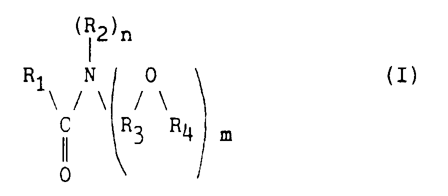
wherein R₁ is the radical of a saturated or unsaturated, linear or branched aliphatic monocarboxylic acid, containing 1 to 24 carbon atoms, optionally substituted with one or more aminic, alcoholic, ketonic, heterocyclic, alicyclic or policyclic groups;
or the radical of an aromatic or heterocyclic monocarboxylic acid. containing 3 to 24 carbon atoms;
or the radical of a saturated or unsaturated aliphatic, araliphatic, aromatic or heterocyclic dicarboxylic acid, containing 2 to 24 carbon atoms, optionally substituted with one or more aminic, alcoholic or ketonic groups;
R₂ is H or a linear or branched C₁-C₈ aliphatic radical, optionally substituted with one hydroxyl group; or an araliphatic or aromatic radical;
R₃ is a linear or branched alkylene chain, containing 1 to 6 carbon atoms, optionally substituted with one or more aryl groups; or an aralkylene chain, containing 7 to 10 carbon atoms;
n is 0 or 1; m is 1 or 2; n + m is 2;
or R₂ and R₃ form together with the nitrogen atom a 4-7 membered ring, optionally substituted with one or more carboxilic groups, and linked to oxygen with a covalent bond;
R₄ is a saccharidic portion bound to the previous molecule portion with a glucosidic linkage, said saccharidic portion being derived from a monosaccharide, disaccharide or trisaccharide, wherein the hydroxil groups of the saccharides are optionally esterified with acetyl, sulphate or phosphate groups, and/or are substituted by one or more aminic groups, optionally N-acylated, thus giving aminosaccharides.

The present invention is also related to the use of the glucosidic derivatives according to the present invention in the treatment of acute and chronic disorders of the nervous system, directly or indirectly associated with cytotoxic events mediated by a prolonged stimulation of Excitatory Amino Acids receptors as well as to pharmaceutical compositions containing the glucosidic derivatives of the present invention as active ingredients.

### DETAILED DESCRIPTION OF THE INVENTION

The following detailed description sets forth the characterization and advantages of the new glucosidic derivatives of N-acyl alkylamines, hereinafter generically referred to as "N-acyl glucamides", active in the prevention of neuronal death phenomena induced by excitotoxic stimulation and useful in disorders connected with supramaximal and prolonged stimulation of EAA receptors. In the derivatives of formula (I), when R₁ is the radical of a monocarboxylic acid, said acid is preferably selected from the group consisting of acetic, caproic, palmitic, oleic, myristic, linoleic, stearic, lauric, nervonic and arachidonic acid, and hydroxyl homologs thereof, e.g. omega-hydroxypalmitic acid, derivatives thereof acrylated on the hydroxyl group, aminic homologs thereof, e.g. gamma-aminobutyric acid (GABA) and gamma-trimethyl-β-hydroxybutyrobetaine, and ketonic homologs thereof, benzoic, trimethoxybenzoic, nicotinic, phenylanthranylic, thioctic and deoxycholic acid.

When R₁ is the radical of a dicarboxylic acid, said acid is preferably selected from the group consisting of fumaric, azelaic, oxalic, succinic, glutaric, pimelic, traumatic, maleic and malonic acid and hydroxyl homologs thereof, phthalic, folic and chromoglycic acid.

The second carboxilic group may be free or may form an ester or an amide. According to a particular aspect of the invention, the second carboxyl may bind a molecule portion identical to that bound to the first carboxyl, thus giving a symmetric bifunctional molecule having formula (II): 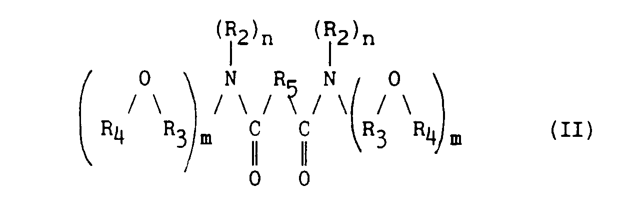
wherein R₂, R₃, R₄, n and m are as defined above and R₅ is the radical of the dicarboxylic acid as defined above.
R₂ is preferably -H, -CH₃, -C₂H₅, -CH₂OH or -C₂H₄OH,
R₃ is preferably ethylene, propylene, -(CH₂)₆-,

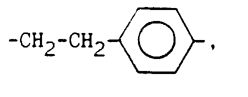

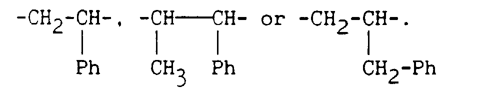

When R₄ is a monosaccharidic portion, it is preferably selected from the group consisting of D- and L-ribose, D- and L-glucose, D- and L-galactose, D- and L-mannose, D-fructose, D- and L-glucosamine, D-galactosamine and D-mannosamine, glucuronic acid and sialic acid (NANA);
when R₄ is a disaccharidic portion, it is preferably selected from the group consisting of lactose, maltose, sialyl-glucose and sialyl-galactose;
when R₄ is a trisaccharidic portion, it is preferably sialyl-lactose. The compounds of the present invention are prepared according to two different schemes.

According to one scheme, an amide of formula (III): 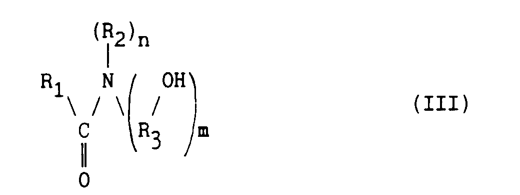 wherein R₁, R₂, R₃, n and m are defined as above, is caused to react with a reactive saccharidic derivative, e.g. α-D-acetobromglucose, in anhydrous and aprotic solvent, preferably acetonitrile or dichloromethane or mixtures thereof, in presence of an equimolar amount of a reaction promoter, e.g. a silver salt, preferably silver trifluoromethane sulphonate, at low temperature. When α-D-acetobromglucose is used, the acetyl groups on the saccaridic hydroxyls are finally removed by alcoholysis.

According to a second scheme:
A) an aminoalcohol of formula R₂HN-R₃-OH, wherein R₂ and R₃ are defined as above, is caused to react with a saccharide in presence of a strong acid, e.g. methanesulphonic acid, under nitrogen atmosphere, optionally in an inert solvent such as DMSO;
B) the reaction product obtained from step (A) is N-acylated with an activated derivative of carboxylic acid, e.g. acyl chloride.

In both cases, the crude products are purified by known methods. Due to their proved biological activities, these compounds may find extensive application in the treatment of both acute and chronic disorders connected with overstimulation excitatory amino acids NMDA receptor, in which mimicking and/or promoting neurotrophic factor effect could be also crucial. Among these, hypoxic-ischaemia, stroke, brain and spinal cord injuries, epilepsy, TIA (transient ischaemic attacks), neurolathyrism and amyotrophic lateral sclerosis, as well as Hungtington's Chorea, Alzheimer's disease and kinds of dementia, either primary or connected with viral diseases, e.g. HIV infection, and Parkinson's disease are of particular significance. In connection with the high distribution of EAA and receptors thereof on an ophthalmic level, said compounds may also be used to treat retinal damages, including anoxia secondary to glaucoma.

Some preparation examples and the characteristics of the compounds according to the present invention are reported hereinbelow for illustrative but not limitative purposes.

The following products, identified by their registered trade marks, are used in the examples: Celite®, Dowex®, LiChrosorb RP-18®, Amberlyst A21 ® and Polysorbate 80 ®.

### Example 1: Preparation of 2-O-(β-D-glucopyranosyl)-N-palmitoylaminoethanol

N-(2-hydroxyethyl)-palmitamide (3.0 g; 10 mmol) was suspended in a solvent mixture consisting of anhydrous dichloromethane (150 ml) and acetonitrile (150 ml) under stirring at -30°C in the dark.

After addition of silver trifluoromethanesulphonate (2.83 g; 11 mmol) and of α-D-acetobromglucose (4.11 g), the mixture was stirred at -30°C for 2 hrs and at 0°C overnight. The obtained suspension was filtered on celite and the filtrate was evaporated to dryness in vacuo. The residue was chromatographed by silica gel eluting with chloroform-methanol-water-ammonia (30%), 85/15/0.5/0.5. The fractions containing the product were combined and evaporated to dryness in vacuo.

The reaction yield was 73%.

The physico-chemical properties of 2-O-(β-D-glucopyranosyl)-N-palmitoylaminoethanol were as follows:

| | |
|---|---|
| physical state | white amorphous solid |
| molecular formula | C₂₄H₄₇NO₇ |
| molecular weight | 461.65 |
| elemental analysis | C=62.44%; H=10.26%; N=3.03%; 0=24.26% |
| solubility in organic solvents | >10 mg/ml in ethanol and DMSO |
| solubility in water | >10 mg/ml in hot water; poorly soluble at room temperature |
| TLC | eluent: chloroform-methanol-water-NH₃ (28%), 80/25/2/1 Rf=0.40 |

### Example 2: Preparation of 2-O-(β-D-glucopyranosyl)-N-acetyl-aminoethanol

N-(2-hydroxyethyl)-acetamide (1.03 g; 10 mmol) was suspended in a solvent mixture consisting of anhydrous dichloromethane (150 ml) and acetonitrile (150 ml) under stirring at -30°C in the dark. After addition of silver trifluoromethanesulphonate (2.83 g; 11 mmol) and of α-D-acetobromglucose (4.11 g), the mixture was stirred at -30°C for 2 hrs and at 0°C overnight. The obtained suspension was filtered on Celite and the filtrate was evaporated to dryness in vacuo. The residue was taken up with anhydrous methanol (50 ml) and added with sodium methoxide (200 mg). The mixture was stirred at room temperature for a period of 30 minutes, added with 50x8 Dowex resin in the anhydrous H+ form (5 g). After stirring for a period of 5 minutes, the resin was separated by filtration, the solution was decolourized with activated carbon and filtered on celite. The filtrate was evaporated to dryness in vacuo. The residue was purified by chromatography in Lichrosorb RP-18 column eluting with water. The fractions containing the product were combined and evaporated to dryness in vacuo. The residue was lyophilized.

The reaction yield was 71%.

The physico-chemical properties of 2-O-(β-D-glucopyranosyl)-N-acetylaminoethanol were as follows:

| | |
|---|---|
| physical state | white amorphous solid |
| molecular formula | C₁₀H₁₉NO₇ |
| molecular weight | 265.27 |
| elemental analysis | C=45.28%; H=7.22%; N=5.28%; 0=42.22% |
| solubility in organic solvents | >10 mg/ml in ethanol and DMSO |
| solubility in water | >10 mg/ml |
| TLC | eluent: chloroform-methanol-water-NH₃ (28%), 80/25/2/1; Rf=0.09 |

### Example 3: Preparation of 2-0-(β-D-glucopyranosyl)-N-dodecanoylaminoethanol

N-(2-hydroxyethyl)-dodecanamide (3.0 g; 10 mmol) was suspended in a solvent mixture consisting of anhydrous dichloromethane (150 ml) and acetonitrile (150 ml) under stirring at -30°C in the dark. After addition of silver trifluoromethanesulphonate (2.83 g; 11 mmol) and of α-D-acetobromglucose (4.11 g), the mixture was stirred at -30°C for 2 hrs and at 0°C overnight. The obtained suspension was filtered on celite and the filtrate was evaporated to dryness in vacuo. The residue was taken up with anhydrous methanol (50 ml) and added with sodium methoxide (200 mg). The mixture was stirred at room temperature for a period of 30 minutes, added with 50x8 Dowex resin in the anhydrous H+ form (5 g). After stirring for a period of 5 minutes, the resin was separated by filtration, the solution was decolourized with activated carbon and filtered on celite. The filtrate was evaporated to dryness in vacuo. The residue was purified by silica gel chromatography eluting with chloroform-methanol-water-ammonia (30%), 90/10/0.5/0.5. The fractions containing the product were combined and evaporated to dryness in vacuo. The reaction yield was 75%.

The physico-chemical properties of 2-0-(β-D-glucopyranosyl)-N-dodecanoylamino ethanol were as follows:

| | |
|---|---|
| physical state | white amorphous solid |
| molecular formula | C₂₀H₃₉NO₇ |
| molecular weight | 405.54 |
| elemental analysis | C=59.24%; H=9.69%; N=3.45%; 0=27.62% |
| solubility in organic solvents | >10 mg/ml in ethanol and DMSO |
| solubility in water | >10 mg/ml in hot water; poorly soluble at room temperature |
| TLC | eluent: chloroform-methanol-water-NH₃ (28%), 80/25/2/1 Rf=0.24 |

### Example 4: Preparation of 2-O-(β-D-galactopyranosyl)-N-palmitoylaminoethanol

N-(2-hydroxyethyl)-palmitamide (3.0 g; 10 mmol) was suspended in a solvent mixture consisting of anhydrous dichloromethane (150 ml) and acetonitrile (150 ml) under stirring at -30°C in the dark. After addition of silver trifluoromethanesuiphonate (2.83 g; 11 mmol) and of α-D-acetobromgalactose (4.11 g), the mixture was stirred at -30°C for 2 hrs and at 0°C overnight. The obtained suspension was filtered on celite and the filtrate was evaporated to dryness in vacuo. The residue was taken up with anhydrous methanol (50 ml) and added with sodium methoxide (200 mg). The mixture was stirred at room temperature for a period of 30 minutes, added with 50x8 Dowex resin in the anhydrous H+ form (5 g). After stirring for a period of 5 minutes, the resin was separated by filtration, the solution was decolourized with activated carbon and filtered on Celite. The filtrate was evaporated to dryness in vacuo. The residue was purified by silica gel chromatography eluting with chloroform-methanol-water-ammonia (30%), 85/15/0.5/0.5. The fractions containing the produdt were combined and evaporated to dryness in vacuo. The reaction yield was 77%.

The physico-chemical properties of 2-0-(β-D-galactopyranosyl)-N-palmitoyl-aminoethanol were as follows:

| | |
|---|---|
| physical state | white amorphous solid |
| molecular formula | C₂₄H₄₇NO₇ |
| molecular weight | 461.65 |
| elemental analysis | C=62.44%; H=10.26%; N=3.03%; 0=24.26% |
| solubility in organic solvents | >10 mg/ml in ethanol and DMSO |
| solubility in water | >10 mg/ml in hot water; poorly soluble at room temperature |
| TLC | eluent: chloroform-methanol-water-NH₃ (28%), 80/25/2/1 Rf=0.34 |

### Example 5: Preparation of 2-0-(β-D-glucopyranosyl)-N-myristoyl-aminoethanol

N-(2-hydroxyethyl)-myristoylamide (2.72 g; 10 mmol) was suspended in a solvent mixture consisting of anhydrous dichloromethane (150 ml) and acetonitrile (150 ml) under stirring at -30°C in the dark. After addition of silver trifluoromethanesulphonate (2.83 g; 11 mmol) and of α-D-acetobromglucose (4.11 g), the mixture was stirred at -30°C for 2 hrs and at 0°C overnight. The obtained suspension was filtered on celite and the filtrate was evaporated to dryness in vacuo. The residue was taken up with anhydrous methanol (50 ml) and added with sodium methoxide (200 mg). The mixture was stirred at room temperature for a period of 30 minutes, added with 50x8 Dowex resin in the anhydrous H+ form (5 g). After stirring for a period of 5 minutes, the resin was separated by filtration, the solution was decolourized with activated carbon and filtered on celite. The filtrate was evaporated to dryness in vacuo. The residue was purified by silica gel chromatography eluting with chloroform-methanol-water-ammonia (30%). 90/10/0.5/0.5. The fractions containing the product were combined and evaporated to dryness in vacuo. The reaction yield was 76%.

The physico-chemical properties of 2-O-(β-D-glucopyranosyl)-N-myristoyl-aminoethanol were as follows:

| | |
|---|---|
| physical state | white amorphous solid |
| molecular formula | C₂₂H₄₃NO₇ |
| molecular weight | 433.59 |
| elemental analysis | C=60.94%; H=10.00%; N=3.23%; 0=25.83% |
| solubility in organic solvents | >10 mg/ml in ethanol and DMSO |
| solubility in water | >10 mg/ml in hot water; poorly soluble at room temperature |
| TLC | eluent: chloroform-methanol-water-NH₃ (28%), 80/25/2/1 Rf=0.28 |

### Example 6: Preparation of 2-0-(β-D-galactopyranosyl)-N-benzoyl-aminoethanol

N-(2-hydroxyethyl)-benzamide (1.65 g; 10 mmol) was suspended in a solvent mixture consisting of anhydrous dichloromethane (100 ml) and acetonitrile (100 ml) under stirring at -30°C in the dark. After addition of silver trifluoromethanesulphonate (2.83 g; 11 mmol) and of α-D-acetobromgalactose (4.11 g), the mixture was stirred at -30°C for 2 hrs and at 0°C overnight. The obtained suspension was filtered on celite and the filtrate was evaporated to dryness in vacuo. The residue was taken up with anhydrous methanol (50 ml) and added with sodium methoxide (200 mg). The mixture was stirred at room temperature for a period of 30 minutes, added with 50x8 Dowex resin in the anhydrous H+ form (5 g). After stirring for a period of 5 minutes, the resin was separated by filtration, the solution was decolourized with activated carbon and filtered on celite. The filtrate was evaporated to dryness in vacuo. The residue was purified by silica gel chromatography eluting with chloroform-methanol-water-ammonia (30%), 80/20/0.5/0.5. The fractions containing the product were combined and evaporated to dryness in vacuo. The reaction yield was 79%.

The physico-chemical properties of 2-0-(β-D-galactopyranosyl)-N-benzoyl-aminoethanol were as follows:

| | |
|---|---|
| physical state | white amorphous solid |
| molecular formula | C₁₅H₂₁NO₇ |
| molecular weight | 327.34 |
| elemental analysis | C=55.04%; H=6.47%; N=4.28%; 0=34.21% |
| solubility in organic solvents | >10 mg/ml in ethanol and DMSO |
| solubility in water | >10 mg/ml |
| TLC | eluent: chloroform-methanol-water-NH₃ (28%), 80/25/2/1 Rf=0.15 |

### Example 7 Preparation of 2-O-(β-D-glucopyranosyl)-N-palmitoyl-aminoethanol A): Preparation of 2-aminoethyl-β-D-glucopyranoside hydrochloride

Anhydrous D-glucose (1.80 g; 10 mmol) and ethanolamine (0.73 g; 12 mmol) were cold solubilized in methanesulphonic acid (3.6 ml). The mixture was heated to room temperature and stirred under nitrogen atmosphere. 24 hours later, the mixture was cooled to 0°C and taken up with cold water (10 ml). Excess methanesulphonic acid was absorbed on Amberlist A21 in the free base form. The resin was separated and the solution was eluted in a column containing 15 ml of anionic exchange resin, 1x8 Dowex. in Cl⁻ form. The eluate was lyophilized and the dry residue was crystallized from ethanol-water.

The reaction yield was 81%.

### B): Preparation of 2-0-(β-D-glucopyranosyl)-N-palmitoyl-aminoethanol

2-aminoethyl-β-D-glucopyranoside hydrochloride obtained from step (A) (1.3 g; 5 mmol) was suspended in cold DMF (30 ml) with triethylamine (1,22 g; 12 mmol). Palmitoyl chloride (1.65 g) was added dropwise over a period of 30 minutes. The resulting solution was heated to room temperature, stirred for a further 20 hours' period and evaporated to dryness. The residue was purified by silica gel chromatography eluting with chloroform-methanol-water-ammonia (30%), 85/15/0.5/0.5. The fractions containing the product were combined and evaporated to dryness in vacuo.

The reaction yield was 92%.

The physico-chemical properties of 2-0-(β-D-glucopyranosyl)-N-palmitoyl-aminoethanol correspond to those of the product obtained in Example 1.

### Example 8: Preparation of 2-0-(β-D-glucopyranosyl)-N-decanoyl-aminoethanol

2.15 g of N-(2-hydroxyethyl)-decanoylamide (10 mmol) were dissolved in 200 ml of an anhydrous mixture of acetonitrile : dichloromethane (1:1). The solution was stirred at -30°C in the dark, with addition of 2.83 g of silver trifluoromethanesulphonate (11 mmol) and 4.11 g di α-D-acetobromglucose (10 mmol). The resulting mixture was then stirred at-30°C for 2 hours, and then at 0°C overnight. The suspension obtained was filtered on celite, the solid residue discarded and the solution evaporated to dryness in vacuo. This residue was dissolved in 50 ml anhydrous methanol, 200 mg sodium methoxide was added and the solution stirred at room temperature for 30 minutes. 5 g of anhydrous sulphonic resin Dowex 50x8 H+ was then added. After 5 minutes the resin was separated and discarded, the solution was decolorized over animal charcoal, filtered on celite and concentrated in vacuo. The resulting crude residue was applied to a silica gel column and eluted using chloroform/methanol/H20/NH3 (30%), (85:15:0.5:0.5). The fractions containing the desired product were collected and concentrated in vacuo. This last residue was dissolved in H2O/isopropanol (3:1) and lyophilized. The final reaction yield was about 80%.

The physico-chemical properties of 2-0-(β-D-glucopyranosyl)-N-decanoyl-aminoethanol were as follows:

| | |
|---|---|
| physical state | white amorphous powder |
| molecular formula | C₁₈H₃₅NO₇ |
| molecular weight | 377.48 |
| elemental analysis | C=57.27%;H=9,35%;N=3.71%;0=29.67% |
| organic solvent solubility | >10mg/ml in ethanol and DMSO |
| water solubility | >10mg/ml |
| TLC | eluent:chloroform/methanol/water/ /NH3(28%), 80:25:2:1) Rf=0.44 |

### Example 9: Preparation of 2-O-(β-D-glucopyranosyl)-N-myristoleoyl-aminoethanol

2.70 g of N-(2-hydroxyethyl)-myristolecylamide (10 mmol) were dissolved under nitrogen atmosphere in 200 ml of an anhydrous mixture of acetonitrile : dichloromethane (1:1). The solution was stirred at -30°C in the dark, with addition of 2.83 g of silver trifluoromethanesulfonate (11 mmol) and 4.11 g di α-D-acetobromglucose (10 mmol). The resulting mixture was then stirred at -30°C for 2 hrs and then at 0°C overnight. The suspension obtained was filtered on celite, the solid residue discarded and the solution evaporated to dryness in vacuo. This residue was dissolved in 50 ml anhydrous methanol, 200 mg sodium methoxide was added and the solution stirred at room temperature for 30 minutes. 5 g of anhydrous sulphonic resin Dowex 50x8 H+ was then added. After 5 minutes the resin was separated and discarded, the solution was decolorized over animal charcoal, filtered on celite and concentrated in vacuo. The resulting crude residue was applied to a silica gel column and eluted using chloroform/methanol (85:15). The fractions containing the desired product were collected and concentrated in vacuo. This last residue was dissolved in H20/isopropanol (3:1) and lyophilized. The final reaction yield was about 78%.

The physico-chemical properties of 2-O-(β-D-glucopyranosyl)-N-myristoleoyl-aminoethanol were as follows:

| | |
|---|---|
| physical state | whitish amorphous powder |
| molecular formula | C₂₂H₄₁NO₇ |
| molecular weight | 431.57 |
| elemental analysis | C=61.23%;H=9.58%;N=3.25%;O=25.95% |
| organic solvent solubility | >10mg/ml in ethanol and DMSO |
| water solubility | poorly soluble |
| TLC | eluent:chloroform/methanol/ /water/acetic acid (80:25:2:1) Rf=0.50 |

### Example 10: Preparation of 2-O-(β-D-glucopyranosyl)-N-oleoyl-aminoethanol

3.26 g of N-(2-hydroxyethyl)-oleoylamide (10 mmol) were dissolved in 200 ml of an anhydrous mixture of acetonitrile: dichloromethane (1:1). The solution was stirred at -30°C in the dark, with addition of 2.83 g of silver trifluoromethanesulphonate (11 mmol) and 4.11 g di α-D-acetobromglucose (10 mmol). The resulting mixture was then stirred at - 30°C for 2 hrs, and then at 0°C overnight. The suspension obtained was filtered on celite, the solid residue discarded and the solution evaporated to dryness in vacuo. This residue was dissolved in 50 ml anhydrous methanol, 200 mg sodium methoxide was added and the solution stirred at room temperature for 30 minutes. 5 g of anhydrous sulphonic resin Dowex 50x8 H+ was then added. After 5 minutes the resin was separated and discarded, the solution was decolorized over animal charcoal, filtered on celite and concentrated in vacuo. The resulting crude residue was applied to a silica gel column and eluted using chloroform/methanol/H2O/NH3 (30%), (85:15:1:0.5). The fractions containing the desired product were collected and concentrated in vacuo. This last residue was dissolved in H₂O/isopropanol (3:1) and lyophilized. The final reaction yield was about 80%.

The physico-chemical properties of 2-0-(β-D-glucopyranosyl)-N-oleoyl-aminoethanol were as follows:

| | |
|---|---|
| physical state | white amorphous powder |
| molecular formula | C₂₆H₄₉NO₇ |
| molecular weight | 487.69 |
| elemental analysis | C=64.03%;H=10.13%;N=2.87%;0=22.97% |
| organic solvent solubility | >10mg/ml in ethanol and DMSO |
| water solubility | poorly soluble |
| TLC | eluent: chloroform/methanol/ /water/NH3 (28%) (80:25:2:1) Rf=0.37 |

### Example 11: Preparation of 2-0-(β-D-glucopyranosyl)-N-stearoyl-aminoethanol

3.28 g of N-(2-hydroxyethyl)-stearoylamide (10 mmol) were dissolved in 200 ml of an anhydrous mixture of acetonitrile:dichloromethane (1:1). The solution was stirred at -30°C in the dark, with addition of 2.83 g of silver trifluoromethanesulphonate (11 mmol) and 4.11 g di α-D-acetobromglucose (10 mmol). The resulting mixture was then stirred at - 30°C for 2 hrs and then at 0°C overnight. The suspension obtained was filtered on celite, the solid residue discarded and the solution evaporated to dryness in vacuo. This residue was dissolved in 50 ml anhydrous methanol, 200 mg sodium methoxide was added and the solution stirred at room temperature for 30 minutes. 5 g of anhydrous sulfonic resin Dowex 50x8 H+ was then added. After 5 minutes the resin was separated and discarded, the solution was decolorized over animal charcoal, filtered on celite and concentrated in vacuo. The resulting crude residue was applied to a silica gel colomn and eluted using chloroform/methanol/H20/ NH3 (30%), (85:15:0.5:0.5). The fractions containing the desired product were collected and evaporated to dryness in vacuo.

The final reaction yield was about 82%.

The physico-chemical properties of 2-0-(β-D-glucopyranosyl)-N-stearoyl-aminoethanol were as follows:

| | |
|---|---|
| physical state | white amorphous powder |
| molecular formula | C₂₆H₅₁NO₇ |
| molecular weight | 489.69 |
| elemental analysis | C=63.77%;H=10.50%;N=2.86%;0=22.87% |
| organic solvent solubility | >10mg/ml in DMSO and hot isopropyl alcohol |
| water solubility | poorly soluble |
| TLC | eluent: chloroform/methanol/water/ /acetic acid, (80:25:2:1) Rf=0.52 |

### Example 12: Preparation of N,N'-bis-[2-0-(β-D-glucopyranosyl)-ethyl]-nonanediamide

2.74 g of N,N'-bis-(2-hydroxyethyl)-nonanediamide (10 mmol) were dissolved in 200 ml of an anhydrous mixture of acetonitrile:dichloromethane (1:1). The solution was stirred at -30°C in the dark, with addition of 5.66 g of silver trifluoromethanesulphonate (22 mmol) and 8.22 g α-D-acetobromglucose (20 mmol). The resulting mixture was then stirred at -30°C for 2 hrs and then at 0°C overnight. The suspension obtained was filtered on celite, the solid residue discarded and the solution evaporated to dryness in vacuo. This residue was dissolved in 50 ml anhydrous methanol, 200 mg sodium methoxide was added and the solution stirred at room temperature for 30 minutes. 5 g of anhydrous sulphonic resin Dowex 50x8 H+ was then added. After 5 minutes the resin was separated and discarded, the solution was decolorized over animal charcoal, filtered on celite and concentrated in vacuo. The resulting crude residue was applied to a silica gel column and eluted using chloroform/methanol/H20/NH3 (30%),(65:35:4:3). The fractions containing the desired product were collected and concentrated in vacuo. This last residue was dissolved in water and lyophilized.

The final reaction yield was about 78%

The physico-chemical properties of N,N"-bis-[2-0-(β-D-glucopyranosyl)-ethyl]-nonanediamide were as follows:

| | |
|---|---|
| physical state | white amorphous powder |
| molecular formula | C₂₅H₄₆N₂O₁₄ |
| molecular weight | 598.64 |
| elemental analysis | C=50.16%;H=7.75%;N=4,68%;0=37.42% |
| organic solvent solubility | >10mg/ml in DMSO |
| water solubility | >10mg/ml |
| TLC | eluent: chloroform/methanol/water/ /NH3 (28%), (65:35:5:3) Rf=0.18 |

### Example 13: Preparation of 2-0-(β-D-glucopyranosyl)-N-arachidonoyl-aminoethanol

3.48 g of N-(2-hydroxyethyl)-arachidonoylamide (10 mmol) were dissolved in 200 ml of an anhydrous mixture of acetonitrile:dichloromethane (1:1) under nitrogen atmosphere. The solution was stirred at -30°C in the dark. with addition of 2.83 g of silver trifluoromethanesulphonate (11 mmol) and 4.11 g of α-D-acetobromglucose (10 mmol). The resulting mixture was then stirred at -30°C for 2 hrs and then at 0°C overnight. The suspension obtained was filtered on celite, the solid residue discarded and the solution evaporated to dryness in vacuo. This residue was dissolved in 50 ml anhydrous methanol under nitrogen atmosphere, 200 mg sodium methoxide was added and the solution stirred at room temperature for 30 minutes. 5 g of anhydrous sulphonic resin Dowex 50x8 H+ was then added. After 5 minutes the resin was separated and discarded, the solution was decolorized over animal charcoal, filtered on celite and concentrated in vacuo. The resulting crude residue was applied to a silica gel column and eluted using chloroform/methanol/H2O/NH3 (30%), (90:10:0.5:0.5). The fractions containing the desired product were collected and concentrated in vacuo. This last residue was dissolved in 95% ethanol and stored under nitrogen atmosphere.

The final reaction yield was about 80%

The physico-chemical properties of 2-0-(β-D-glucopyranosyl)-N-arachidonoyl-aminoethanol were as follows:

| | |
|---|---|
| physical state | whitish amorphous powder |
| molecular formula | C₂₈H₄₇NO₇ |
| molecular weight | 509.69 |
| elemental analysis | C=65.98%;H=9.30%;N=2.75%;0=21.97% |
| organic solvent solubility | >10mg/ml in ethanol and DMSO |
| water solubility | poorly soluble |
| TLC | eluent: chloroform/methanol/water/ /NH3(28%),(80:25:2:1) Rf=0.55 |

### Example 14: Preparation of 2-0-(α-D-glucopyranosyl)-N-dodecanoyl-aminoethanol

3.90 g of β-D-glucose pentacetate (10 mmol) and 2.5 g hydrazine acetate were dissolved in 70 ml of anhydrous dimethylformamide. The solution was stirred at 50°C for 3 hrs and then evaporated to dryness in vacuo. This residue, made up of 2,3,4,6-tetracetyl-D-glucose, was dissolved in 50 ml of anhydrous dichloromethane with addition of 5 ml of trichloroacetonitrile and 0.5 ml of 1.8-diazabicyclo-[5,4,0]-7-undecene; this solution was stirred at room temperature for 1 hour and then washed twice with 25 ml H20, dryed over anhydrous sodium sulphate and evaporated to dryness in vacuo. The residue was dissolved in 50 ml or anhydrous diethyl ether with addition of 2.44 g of N-(2-hydroxyethyl)-dodecanoylamide (10 mmol) and 2.22 g of trimethylsilyl trifluoromethanesulfonate (10 mmol). The resulting mixture was then stirred at 0°C for 3 hrs and then evaporated to dryness in vacuo. This residue was dissolved in 50 ml anhydrous methanol, 200 mg sodium methoxide was added and the solution stirred at room temperature for 30 minutes. 5 g of anhydrous sulphonic resin Dowex 50x8 H+ was then added. After 5 minutes the resin was separated and discarded, the solution was decolorized over animal charcoal, filtered on celite and concentrated in vacuo. The resulting crude residue was applied to a silica gel column and eluted using chloroform/methanol/H20/NH3 (30%).(85:15:0.5:0.5). The fractions containing the desired product were collected and concentrated in vacuo. This last residue was dissolved in H20/isopropanol (3:1) and lyophilized.

The final reaction yield was about 70%.

The physico-chemical properties of 2-O-(α-D-glucopyranosyl)-N-dodecanoyl-aminoethanol were as follows:

| | |
|---|---|
| physical state | white amorphous powder |
| molecular formula | C₂₀H₃₉NO₇ |
| molecular weight | 405.53 |
| elemental analysis | C=59.24%;H=9.69%;N=3.45%;O=27.62% |
| organic solvent solubility | >10mg/ml in ethanol and DMSO |
| water solubility | >10mg/ml in hot water |
| TLC | eluent: chloroform/methanol/H20/ /acetic acid, (80:25:2:1) Rf=0.46 |

### BIOLOGICAL ACTIVITY

The protective effect against Excitatory Amino Acids, the neurotrophic activity and the capability to reduce serotonin release by antigen stimulated RBL-2H3 cells of the compounds according to the present invention was evaluated in vitro:
Example 1: 2-O-(β-D-glucopyranosyl)-N-palmitoyl-aminoethanol
Example 2: 2-O-(β-D-glucopyranosyl)-N-acetyl-aminoethanol
Example 3: 2-O-(β-D-glucopyranosyl)-N-dodecanoyl-aminoethanol
Example 4: 2-O-(β-D-galactopyranosyl)-N-palmitoyl-aminoethanol
Examole 5: 2-O-(β-D-glucopyranosyl)-N-miristoyl-aminoethanol
Example 6: 2-O-(β-D-galactopyranosyl)-N-benzoyl-aminoethanol
Example 8: 2-O-(β-D-glucopyranosyl)-N-decanoyl-aminoethanol
Example 9: 2-O-(β-D-glucopyranosyl)-N-myristoleyl-aminoethanol
Example 10: 2-O-(β-D-glucopyranosyl)-N-oleoyl-aminoethanol
Example 11: 2-O-(β-D-glucopyranosyl)-N-stearoyl-aminoethanol

### a) Evaluation of neuroprotective effect against Excitatory Amino Acids-induced neuronal death in vitro

### Materials and Methods

### Compounds solubilization

The compounds being evaluated, referred to as No. 1, 2, 3, 4, 5, 9 and 10 (with reference to the molecules whose syntheses have been described by way of example), Diltiazem, MK 801 and monosialoganglioside GM1, were solubilized in Locke's solution and added to the culture mediums at the desired concentrations according to different treatment schedule indicated in the Tables, with a view to checking:
- dose-effect relationship;
- time-effect relationship vs. incubation time (start and duration) and exposure to neurotoxin.

### Cultures preparation

Granule cell cultures were prepared from post natal day 8-9 mouse Balb-6 cerebellum. The cells were suspended in EBM + 2mM L-glutamine, 100 U/ml penicillin, 50 µg/l gentamicin, 25 mM KCl and 10% fetal calf serum, plated on polylysine substrate, cultured for 8-10 days, and exposed to: i) L-glutamic acid (100 µM) at room temperature for 30-40 min. ii) L-glutamic acid (500 µM) at room temperature for 5 min.

The compounds being evaluated were incubated at different times (2 hrs before and at the same time as exposure) or for different times ranging from 5 to 60 minutes after exposure to L-glutamic acid. The cultures were then brought back to the original medium and the number of survived cells was assessed by colorimetric method (MTT) 24 hours after excitotoxic stimulation.

### Results

All tested compounds according to the present invention, though to a different degree, are able to protect granule cells against L-glutamic acid thereinafter referred to as Glu) neurotoxicity, according to a dose-response relationship, as shown in Table 1. Furthermore said compounds do not display intrinsic cytotoxicity.

It is noteworthy to note that these compounds show a time-effect relationship, as shown in Table 2, and can act even after a very short incubation time (see Table 3) and even if added after exposure to an excitotoxic stimulus of different intensity: Glu 100 µM for 30 min (see Table 4) or Glu 500 µM for 5 min (see Table 5 and 6). N-acyl alkylamine glucosidic derivatives are cytoprotective even when incubated after Glu removal. Conversely, the receptor agonist MK 801 is protective, at least partially, only after co-treatment, while GM1, under said particular experimental condition of cytotoxicity, does not show any significant action, like the calcium antagonist Diltiazem (Tables 5).

Said experimental evidences show that: i) like the compounds whose cytoprotective effect is known and which, therefore, have been utilized as controls (MK 801, GM1 and Diltiazem), the new N-acyl glucamides according to the present invention are effective under pre-treatment and co-treatment, indicating their capability to affect cytotoxicity induction mechanisms mediated by EAA receptor; ii) unlike MK 801, GM1 and Diltiazem, the N-acyl glucamides of the invention are effective also when added after Glu (post-treatment) (e.g. compound No. 3 in Table 4), indicating that these compounds can also affect the events occurring downstream of glutamate receptor activation, such as the functionality of membrane enzymes, thus concurring to limit calcium ions influx into the ceil.

**Table 1:**

| | | | |
|---|---|---|---|
| dose-response effect against cellular death induced by Glu (100 µM at room temperature for 40 min), of compounds No. 1 and No. 3, administered at the same time as exposure to Glu (co-treatment), and of compounds No. 2 and No. 4, administered 2 hrs before exposure and being in the culture at the time of exposure to Glu (pre-treatment + co-treatment). | | | |

| **Compound** | **Concentration (µM)** | **Survived Cells** | **(%) EC 50 (µM)** |
|---|---|---|---|
| Glu Ex. No.1 | | 19 | |
| | 10 | 22 | |
| | 25 | 35 | |
| | 50 | 48 | 65 |
| | 100 | 67 | |
| | 200 | 90 | |
| Ex. No.2 | 50 | 25 | |
| | 100 | 35 | 50 |
| | 200 | 50 | |
| Ex. No.3 | 10 | 22 | |
| | 25 | 24 | |
| | 50 | 62 | 30 |
| | 100 | 80 | |
| | 200 | 78 | |
| Ex. No.4 | 10 | 24 | |
| | 25 | 42 | |
| | 50 | 48 | 34 |
| | 100 | 78 | |
| | 200 | 70 | |

Remark: EC 50 stands for "Effective Concentration 50", i.e. the concentration at which the effect is 50% of the maximal value.

**Table 2:**

| | | | | |
|---|---|---|---|---|
| time course of the antiexcitotoxic effect of N-acyl glucamides according to the present invention. Neurotoxicity was induced by exposure to Glu (100 µM) for 40 min at room temperature. Compounds No. 1 (100 µM), No. 2, No. 3 and No. 4 (200 µM) were added, with respect to the neurotoxin exposure: 2 hrs before and then removed (pre.); at the same time (co.); or combining the two protocols (pre. + co.). Cell survival was assessed by colorimetry after 24 hrs. | | | | |

| | **survived cells (%)** | | | |
|---|---|---|---|---|
| **Compound** | **untreated** | **pre.** | **co.** | **pre.+ co.** |
| Glu + No.1 | 15 | 44 | 57 | 35 |
| Glu + No.2 | 14 | 25 | 22 | 36 |
| Glu + No.3 | 15 | 55 | 23 | 76 |
| Glu + No.4 | 24 | 22 | 71 | 79 |

**Table 3:**

| | | | | |
|---|---|---|---|---|
| time course of the antiexcitotoxic effect of N-acyl glucamides according to the present invention. Neurotoxicity was induced by exposure of the cells to Glu (500) µM for 5 min at room temperature. Compound No. 3 was added, at a concentration of 200 µM, immediately after Glu removal, and incubated for 15, 30 or 60 minutes. After washing, cultures were plated again in the medium: cell survival was measured after 24 hrs. | | | | |

| | **survived cells (%)** | | **after incubation of** | |
|---|---|---|---|---|
| | **0 min** | **15 min** | **30 min** | **60 min** |
| Glu | 45 | | | |
| Glu + No.3 | | 50 | 64 | 76 |

**Table 4:**

| | |
|---|---|
| protective effect against neurotoxicity of N-acyl glucamides according to the present invention, added gradually after the excitotoxic stimulus. Neurotoxicity was induced by exposure to Glu (100 µM) for 30 min at room temperature. Compound No. 3 was added at various times (0, 5, 10, 15, 30 min) from the beginning of the 30 minutes' exposure to Glu. Cell survival was measured by colorimetry after 24 hours. | |

| **Time (min) of compound incubation** (during exposure to Glu) | **survived cells (%)** |
|---|---|
| nil | 12 |
| 0-30 | 85 |
| 5-30 | 65 |
| 10-30 | 46 |
| 15-30 | 34 |
| 30 | 16 |

**Table 5:**

| | | | | | |
|---|---|---|---|---|---|
| protective effect against neurotoxicity induced by Glu (500 µM for 5 min at room temperature) of compound No. 3 (200 µM) in comparison with Diltiazem (10 µM) MK 801 (10 µM) and monosialoganglioside GM1 (100 µM), added at various times (0, 15, 30, 60 min) after Glu removal: A: compound No. 3, monosialoganglioside GM1, MK 801 and Diltiazem were incubated for 24 hrs. B: compound No. 3 was incubated for 60 min. Cell survival was measured by colorimetry after 24 hrs. | | | | | |

| addition time of the compounds after Glu removal | | | | | |
|---|---|---|---|---|---|
| | | 0 min | 15 min | 30 min | 60 min |
| | | survived cells (%) | | | |
| A | Glu 43 | | | | |
| | Glu + No.3 | 81 | 67 | 72 | 50 |
| | Glu 37 | | | | |
| | Glu + MK 801 | 66 | 32 | 30 | - |
| | Glu 49 | | | | |
| | Glu + Diltiazem | 35 | - | - | - |
| | Glu 34 | | | | |
| | Glu + GM1 | 31 | - | - | - |
| B | Glu 46 | | | | |
| | Glu + No. 3 | | 93 | 90 | 34 |

**Table 6:**

| | |
|---|---|
| neuroprotective effect of the N-acyl glucamides according to the present invention (Examples 5, 9 and 10), which were added at a concentration of 100 µM for 60 minutes, 15 minutes after glutamate (500 µM for 5 minutes at room temperature). Cell survival was evaluated after 24 hrs by MTT assay. | |

| Treatment | Cell survival (%) |
|---|---|
| Glutamate | 42 |
| Glutamate + Example 5 | 100 |
| Glutamate + Example 9 | 70 |
| Glutamate + Example 10 | 66 |

### b) Evaluation of neurorophic effect: protection against apoptotic neuronal cell death

### Materials and Methods

### Solubilization of the compounds

The compounds as in Ex.3, 5, 8, 9 and 11 and GM1 were solubilized in DMSO 0.2% and added to the cells at the desired concentration.

### Cell culture preparation

Mouse (Balb 6, 8-9 post-natal day) cerebellar granule cells were plated in basal Eagle medium (BEM) + 10% fetal calf serum + 25 mMHcl + 2 mM glutamine + 100 µg/ml gentamicine dishes and coated with poly/L lisine. Culture medium was replaced with serum-free medium 6-9 days after plating. Cells were washed and maintained in serum free medium supplemented with KCl 25mM (control) or KCl 5mM ± examined compounds (S.R. D'Mello et al., *Induction of apoptosis in cerebellar granule neurons by low potassium: inhibition of death by insulin-like growth factor I and cAMP,* 1993, PNAS 90:10989-10993). Neuronal cell survival was measured 24 hrs later by MTT assay.

### Results

All examined compounds of the invention are capable to reduce neuronal apoptosis, as show in Table 7, displaying a GM1 similar potency, GM1 being a well known trophic drug.

**Table 7:**

| | |
|---|---|
| protection against apoptotic neuronal death induced by low potassium (KCl 5mM) exerted by compounds Ex.3, 5, 8, 9 and 11. The compounds were solubilized in DMSO 0.2% and used at the final concentration of 100 µM. | |

| **Treatment** | **Cell survival (%)** |
|---|---|
| KCl 25 mM | 100 |
| KCL 5 mM | 30 |
| KCl 5 mM + Example 3 | 73 |
| KCl 5 mM + Example 5 | 79 |
| KCl 5 mM + Example 8 | 51 |
| KCl 5 mM + Example 9 | 45 |
| KCl 5 mM + Example 11 | 51 |
| KCl 5 mM + GM1 | 78 |

### c) Evaluation of the anti-inflammatory effect: down-modulation of RBL-2H3 cells

### Materials and Methods

### Solubilization of the compounds

The compounds under evaluation as per Ex. 1, 3, 4, and 9 were dissolved in DMSO 0.2%, while the compound of Ex. 6 was dissolved in DMSO 1%, and they were added at the desired concentration of 100 µM or 60 µM.

### Preparation of RBL cell culture

Rat basophil leukemia cells of the secreting subline 2H3 were grown in stationary cultures at 37°C in Eagle's minimal essential medium supplemented to contain 2mM L-glutamine, 100 IU/ml penicillin and 20% (v/v) heat-inactivated fetal calf serum (FCS). Cells were passed twice a week.

### [3H]serotonin release assay

A mouse monoclonal IgE which is specific for dinitrophenol (DNP) haptens (clone SPE-7; Sigma) was used. Dinitrophenylated human serum albumin (DNP-HSA) was employed as the triggering agent in these experiments. The level of conjunction was 30-40 moles of DNP per mole of albumin (Sigma). Prior to a release assay, RBL-2H3 cells were detached in 0.5 mM EDTA/phosphate-assay, RBL-2H3 cells were detached in 0.5 mM EDTA/phosphate-buffered saline (pH 7.2) (PBS) and replated in 6-mm diameter 96-well microplates (Falcon), with each well containing 1x10⁵ cells in 100 µl of RPMI-1640 medium supplemented with 50 µg/ml gentamycin, 10% FCS, and 0.1 µCi of [3H]serotonin ([5-1,2-3H(N)]hydroxytryptamine binoxalate) (26.4 Ci/mmol) (New England Nuclear). After 18 hrs incubation at 37°C, this medium was replaced with 100 µl per well of 0.3 µg/ml anti-DNP IgE in PIPES buffer (25 mM PIPES, 100 mM NaCl, 5 mM KCl, 0.4 mM MgCl2, 1 mM CaCl2, 5.6 mM glucose, pH 7.1) to sensitize the cells (lh, 37°C). The IgE solution was then replaced with 100 µl per well of a prewarmed solution containing 0.1 µg/ml DNP-HSA in the same PIPES buffer. Initial studies showed these concentrations to provide for maximal release (15-30 % net release) of [3H]serotonin. The examined compound was added to the culture wells at this time. Incubation was continued for a further 15 min (37°C). The culture supernatants were then collected in Eppendorf tubes, centrifuged (4 min, 3000 rpm) and 50 µl aliquots counted by standard liquid scintillation techniques. The cellular contents of each well were solubilized with 100 µl of 1% Triton X-100 in PBS, and a 50 µl aliquot counted as above. The percent of [3H]serotonin release was calculated.

Background (spontaneous) release was normally < 5 % of the total radioactivity incorporated, and was always substracted from the stimulated release value ("net" release).

### Results

All tested coumpounds of the present invention are capable to inhibit [3H]serotonin release from DNP-HSA stimulated mast cells when applied in co-treatment, thus indicating their capability to negatively modulate mast cell activation, as shown in Table 8.

**Table 8:**

| | |
|---|---|
| activity of the compounds of Ex. 1, 3, 4, 6 and 9 in negatively modulation of mast cell activation. The activity was measured as percentage of serotonin net release inhibition. | |

| **Compound** | **% of inhibition** |
|---|---|
| Ex. 1 | 57 |
| Ex. 3 | 72 |
| Ex. 4 | 72 |
| Ex. 6 | 54 |
| Ex. 9 | 37 |

On the whole, the experimental evidences suggest that the N-acyl glucamides according to the present invention are capable, even at low concentrations, of exerting a specific protective action against the cytotoxicity of excitatory amino acids on neuronal cells.

Said N-acyl glucamides also possess a trophic activity since they are able to limit apoptotic neuronal death, a form of programmed cell death which has clearly been shown to occurr in sympathetic neurons following NGF deprivation (S.R. D'Mello, cit. ref.).

In addition, the N-acyl glucamides according to the present invention display anti-inflammatory properties, as demonstrated by their capability to negatively modulate RBL cell response to an exogenous stimulus.

Particular emphasis has to be given to the outstanding therapeutic importance of N-acyl glucamides, in that in the EAA toxicity experimental models i) they are characterized by an extremely prompt effect and ii) they are active when administered not only before or at the same time as, but also after exposure to excitatory amino acids, thus affecting not only the neurotoxic mechanisms mediated by the activation of glutamate or kainate receptor, but also the mechanisms following receptor stimulation. In addition it is important to underline iii) their capability to limit apoptotic neuronal death associated with trophic factor deficiency also when added during trophic support deprivation and also iv) their anti-edema/anti-inflammatory effect when added at the same time as activating stimulus.

Furthermore, the compounds do not show any significant intrinsic cytotoxicity.

Therefore, the derivatives described herein can be advantageously used in the treatment of both acute and chronic phase of CNS disorders of humans and animals, whose etiology/evolution is associated with overstimulation of excitatory amino acids receptors.

It should be emphasized that currently available therapies are of limited value in the acute phase of diseases, such as cerebral ictus, which has an extremely narrow "therapeutic window" in terms of the time frame during which neuronal cell death can be limited by pharmacological intervention. In this context, it is of great significance that the N-acyl glucamides of the present invention are able to act not only within these very short times, as already mentioned above, but also later on, when damage has been established. Furthermore, since said new derivatives, on the basis of the experimental evidences herein described, do not act as NMDA-receptor competitive or non-competitive inhibitors, they may be effectively used also to treat chronic diseases, such as Hungtington's Chorea, Parkinson's disease and all types of dementia conditions, without negatively affecting the neuronal plasticity process induced by physiological receptor stimulation and thus not worsening the clinical outcome, as the NMDA-receptor inhibitors cause.

In this regard, both the trophic and anti-inflammatory effect displayed by the N-acyl glucamides of the present invention are of particular therapeutic importance since said neurodegenerative disorders are frequently associated both to trophic factor deficiency and to inflammatory conditions.

It may be useful to remind that the drug administration doses, times and ways are to be diversified depending on the disease type, stage and severity. A distinction is to be made between the acute stage treatment of acute conditions hypoxic-ischaemia (stroke, brain and spinal cord injuries, hypoglycaemia, cerebral hypoxic states associated with cardiovascular surgery) or diseases inducing an immediate and massive glutamic acid release, such as epilepsy as well as transient ischaemic attacks (TIA) -which also are prodromic to ictus-, neurolathyrism, and the treatment of chronic degenerative diseases, such as Huntington's Chorea, Parkinson's and Alzheimer's diseases and dementia, either primary or following viral infections, such as acquired immunodeficiency syndrome (AIDS dementia complex) and amyotrophic lateral sclerosis.

Furthermore, primary retinic diseases even of anoxic nature or associated with eye hypertension, e.g. glaucoma, are to be taken into consideration. All aforesaid diseases may be conveniently treated by the systemic administration of the claimed compounds, both orally and parenterally, but even by topical and transdermal routes.

The therapeutic dose varies depending on the patient's age and weight as well as on the type of disease from 0.1 to 100 mg/kg/die, preferably from 1 to 30 mg/kg/die, over variable periods depending on the disease, in any case for at least 30 days.

The pharmaceutical compositions are inclusive of all formulations including pharmaceutically acceptable excipients, suitable for the administration of active ingredients according to the present invention in the forms best suited to the disease to be treated and, in any case, making the active ingredient as bioavailable as possible. In particular, the solutions are for intravenous, subcutaneous and intramuscular administration, and for ophthalmic treatment particularly in the form of collyria or ointments. As concerns the formulations per os granular powders, tablets, pills and capsules are preferred.

### Example 1: Vials for injection

Every vial contains:

| | |
|---|---|
| Active ingredient Ex. No. 3 | 10 mg |

| Excipients: | |
|---|---|
| mannitol | 12 mg |
| sodium metabisulphite | 1.3 mg |
| benzyl alcohol | 80 mg |
| propylene glycol | 400 mg |
| sodium hydroxide | q.s. to pH 7.4 |
| water for injectable formulations | q.s. to 2 ml |

### Example 2: Tablets

One tablet contains:

| | |
|---|---|
| Active ingredient Ex. No. 3 | 50 mg |

| Excipients: | |
|---|---|
| dibasic dihydrated calcium phosphate | 135.2 mg |
| microgranular cellulose | 36 mg |
| maize starch | 7.2 mg |
| magnesium stearate | 1.8 mg |
| hydrogenated vegetable oil | 1.2 mg |
| precipitated silica | 0.6 mg |
| hydroxypropylmethylcellulose | 4.68 mg |
| titanium dioxide | 0.32 mg |

### Example 3: Collyrium

Every bottle contains:

| | |
|---|---|
| Active ingredient Ex. No. 6 | 25 mg |

| Excipients: | |
|---|---|
| borax | 15 mg |
| boric acid | 75 mg |
| polysorbate 80 | 15 mg |
| lactose | 80 mg |
| phenol | 3.9 mg |
| disodium edetate | 5 mg |
| water for injectable formulations | q.s. to 5 ml |

In conclusion, the compounds of the present invention, when adequately formulated, may be conveniently used in human and animal therapy for the treatment of the diseases in which the acute and chronic neurological damages are directly or indirectly due to or are associated with the excitotoxicity of Excitotoxic Amino Acids. Said diseases are e.g.: hypoxic and/or ischaemic cerebral insults, acute or recurrent and transient attacks, such as TIA; brain or spinal spinal cord injuries; neurodegenerative disorders of unknown etiology (Alzheimer's and Parkinson's diseases. Huntington's Chorea, amyotrophic lateral sclerosis), or the disorders derived from neuronal disturbances following epilepsy and severe hypoglycaemic states; neurological complications whose primary cause is an infection (e.g. HIV dementia complex); neurological complications following hypoxic states caused by cardiovascular surgery or heart failure; diseases pertaining to the retina and in general to the visual system, as well as pertaining other cranial nerves, e.g. the nerves efferent to the hearing system. The neurological diseases associated with excitotoxic damage on dismetabolic or toxic agents, e.g. neurolathyrism, are also to take into consideration.

## Claims

1. Saccharidic derivatives of N-acvl alkvlamines of formula (I): 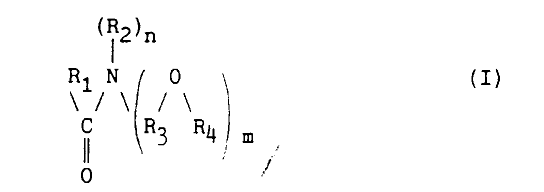
where R₁ is the radical of a saturated or unsaturated, linear or branched aliphatic monocarboxylic acid, containing 1 to 24 carbon atoms, optionally substituted with one or more aminic, alcoholic, ketonic, heterocyclic, alicyclic or policyclic groups;
or is the radical of an aromatic or heterocyclic monocarboxylic acid, containing 3 to 24 carbon atoms;
of the radical of a saturated or unsaturated aliphatic, aralaliphatic, aromatic or heterocyclic dicarboxylic acid, containing 2 to 24 carbon atoms, optionally substituted with one or more aminic, alcoholic or ketonic groups;
R₂ is H or a linear or branched C₁-C₈ aliphatic radical, optionally substituted with one hydroxyl group; or an araliphatic or aromatic radical; R₃ is a linear or branched alkylene chain, containing 1 to 6 carbon atoms, optionally substituted with one or more aryl groups; or an aralkylene chain, containing 7 to 10 carbon atoms;
n is 0 or 1; m is 1 or 2; n + m is 2;
or R₂ and R₃ form together with the nitrogen atom a 4-7 membered ring, optionally substituted with one or more carboxilic groups, and linked to oxygen with a covalent bond;
R₄ is a saccharidic portion bound to the previous molecule portion with a glucosidic linkage, said saccharidic portion being derived from a monosaccharide, a disaccharide or a trisaccharide, wherein the hydroxil groups of the saccharides are optionally esterified with acetyl, sulphate or phosphate groups, and/or are substituted by one or more aminic groups, optionally N-acylated,
with the exclusion of the following compounds;
- 2-O-(β-D-glucopyranosyl)-N-decanoyl-aminoethanol;
- 2-O-(β-D-glucopyranosyl)-N-dodecanoyl-aminoethanol;
- 2-O-(β-D-glucopyranosyl)-N-myristoyl-aminoethanol;
- 2-O-(β-D-glucopyranosyl)-N-palmitoyl-aminoethanol;
- 2-O-(β-D-glucopyranosyl)-N-decanoyl-3-aminopropanol;
- 2-O-(β-D-glucopyranosyl)-N-dodecanoyl-3-aminopropanol;
- 2-O-(β-D-glucopyranosyl) -N-myristoyl-3-aminopropanol;
- 2-O-(β-D-glucopyranosyl)-N-palmitoyl-5-aminopentanol;
- 2-O-(α-D-glucopyranoeyl)-N-decanoyl-aminoethanol;
- 2-O-(α-D-glucopyranosyl)-N-decanoyl-3-aminopropanol.

2. The saccharidic derivatives according to claim 1, wherein said monocarboxylic acid is selected from the group consisting of acetic, caproic, palmitic, oleic, myristic, linoleic, stearic, lauric, nervonic, arachidonic, omega-hydroxy-palmitic, gamma-aminobutyric, gamma-trimethyl-β-hydroxybutyrobetaine and the derivatives thereof acylated on the hydroxyl group, benzoic, trimethoxybenzoic, nicotinlc, phenylanthranylic, thioctic and deoxycholic acid.

3. The saccharidic derivatives according to claim 1, wherein said dicarboxylic acid is selected from the group consisting of fumaric, azelaic, oxalic, succinic, glutaric, pimelic, traumatic, maleic and malonic acid and hydroxyl homologues thereof, phthalic, folic and chromoglycic acid.

4. The saccharidic derivatives according to claim 3, wherein the second carboxyl of said dicarboxylic acid is functionalized to give an ester or an amide.

5. The saccharidic derivatives according to claim 3, wherein the second carboxyl of said dicarboxylic acid binds a molecule portion identical to that bound to the first carboxyl, with formation of a symmetric molecule of formula (II): 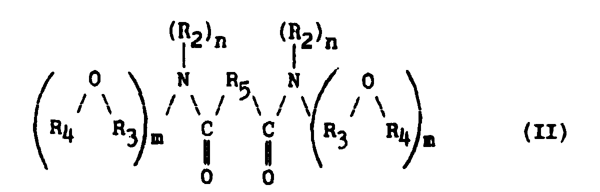 wherein R₁, R₂, R₃, R₄, m and n are as defined in claim 1 and R₅ is the hydrocarbon divalent radical of said dicarboxylic acid.

6. The saccharidic derivatives according to claims 1 to 5, wherein R₂ is H, n is 1 and m is 1.

7. The saccharidic derivatives according to claims 1 to 5, wherein R₂ is selected from the group consisting of -CH₃, -C₂H₅, -CH₂OH and -C₂H₄OH; n is and m is 1.

8. The saccharidic derivatives according to claims 1 to 7, wherein R₃ is ethylene, propylene, -(CH₂)₆-.

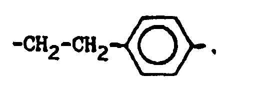

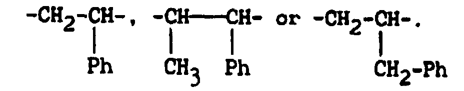

9. The saccharidic derivatives according to claims 1 to 8, wherein R₄ is a monosaccharidic portion selected from the group consisting of D- and L-ribose, D- and L-glucose, D- and L-galactose, D- and L-mannose, Dfructose, D- and L-glucosamine, D-galactosamine, D-mannosamine, glucuronic acid and sialic acid.

10. The saccharidic derivatives according to claims 1 to 8, wherein R₄ is a disaccharidic portion selected from the group consisting of lactose, maltose, sialyl-glucose and sialyl-galactose.

11. The saccharidic derivatives according to claims 1 to 8, wherein R₄ is the trisaccharidic portion of sialyl-lactose.

12. 2-O-(β-D-glucopyranosyl)-N-acetyl-aminoethanol.

13. 2-O-(β-D-galactopyranosyl)-N-palmitoyl-aminoethanol.

14. 2-O-(β-D-galactopyranosyl)-N-benzoyl-aminoethanol.

15. 2-0-(β-D-glucopyranosyl)-N-myristoleyl-aminoethanol.

16. 2-O-(β-D-glucopyranosyl)-N-oleoyl-aminoethanol.

17. 2-O-(β-D-g1ucopyranosyl)-N-stearoyl-aiinoethancl.

18. Process for the preparation of the saccaridic derivatives according to claim 1, wherein an amide of formula: 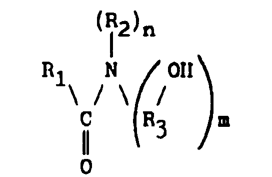 where R₁, R₂, R₃, n and m are defined as in claim 1, is caused to react with a reactive saccharidic derivative, in anhydrous and aprotic solvent, in presence of equimolar amounts of a reaction promoter, at low temperature.

19. The process according to claim 18, wherein said solvent is acetonitrile or dichloromethane or mixtures thereof.

20. The process according to claim 18, wherein said reaction promoter is a silver salt.

21. The process according to claim 20, wherein said silver salt is silver trifluoromethanesulphonate.

22. Process for the preparation of the saccaridic derivatives according to claim 1, comprising the following steps:
A) an aminoalcohol of formula R₂-NH-R₃-OH, wheroin R₂ and R₃ are defined as in claim 1, is caused to react with a saccharide, in presence of a strong acid, under nitrogen atmosphere;
B) the reaction product obtained from step (A) is N-acylated with an activated derivative of R₁-COOH, wherein R₁ is defined as in claim 1.

23. The process according to claim 22, wherein step (A) is carried out in an inert solvent.

24. The process according to claim 23, wherein in step (A) said strong acid is methanesulphonic acid.

25. Pharmaceutical compositions comprising as the active principle at least one saccaridic derivative of N-acyl alkylamines of formula (I): 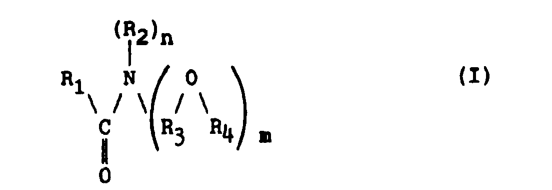
where R₁ is the radical of a saturated or unsaturated, linear or branched aliphatic monocarboxylic acid, containing 1 to 24 carbon atoms, optionally substituted in the aliphatic chain with one or more aminic, alcoholic, ketonic, heterocyclic, alicyclic or policyclic groups;
or is the radical of an aromatic or heterocyclic monocarboxylic acid, containing 3 to 24 carbon atoms;
of the radical of a saturated or unsaturated aliphatic, aralaliphatic, aromatic or heterocyclic dicarboxylic acid, containing 2 to 24 carbon atoms, optionally substituted with one or more aminic, alcoholic or ketonic groups;
R₂ is H or a linear or branched C₁-C₈ aliphatic radical, optionally substituted with one hydroxyl group: or an araliphatic or aromatic radical;
R₃ is a linear or branched alkylene chain, containing 1 to 6 carbon atoms, optionally substituted with one or more aryl groups; or an aralkylene chain, containing 7 to 10 carbon atoms;
n is O or 1; m is 1 or 2; n + m is 2;
or R₂ and R₃ form together with the nitrogen atom a 4-7 membered ring, optionally substituted with one or more carboxilic groups, and linked to oxygen with a covalent bond;
R₄ is a saccharidic portion bound to the previous molecule portion with a glucosidic linkage, said saccharidic portion being derived from a monosaccharide, a disaccharide or a trisaccharide, wherein the hydroxil groups of the saccharides are optionally esterified with acetyl, sulphate or phosphate groups, and/or are substituted by one or more aminic groups, optionally N-acylated;
in combination with suitable excipients and/or diluents.

26. The pharmaceutical compositions according to claim 25, administrable by oral or parenteral route.

27. The pharmaceutical compositions according to claim 26, orally administrable in the form of granular powders, tablets, pills or capsules.

28. The pharmaceutical compositions according to claim 26, intravenously, subcutaneously or intramuscularly administrable.

29. The pharmaceutical compositions according to claim 25, administrable by topical or transdermal route.

30. The pharmaceutical compositions according to claim 25, suitable for ophtalmic use in the form of collyria or ointments.

31. The pharmaceuticai compositions according to claim 25, wherein said active principle is administered at a dosage ranging from 0.1 to 100 mg/kg/day, for at least 30 days.

32. The pharmaceutical compositions according to claim 31, wherein said dosage ranges from 1 to 30 mg/kg/day.

33. A saccharidic derivative of N-acyl alkylamines of formula (I): 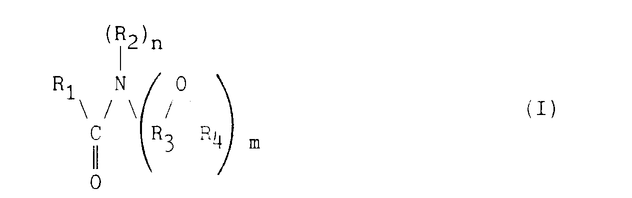
where R₁ is the radical of a saturated or unsaturated, linear or branched aliphatic monocarboxylic acid, containing 1 to 24 carbon atoms, optionally substituted in the aliphatic chain with one or more aminic, alcoholic, ketonic, heterocyclic, alicyclic or policyclic groups; or is the radical of an aromatic or heterocyclic monocarboxylic acid, containing 3 to 24 carbon atoms;
or the radical of a saturated or unsaturated aliphatic, aralaliphatic, aromatic or heterocyclic dicarboxylic acid, containing 2 to 24 carbon atoms, optionally substituted with one or more aminic, alcoholic or ketonic groups;
R₂ is H or a linear or branched C₁-C₈ aliphatic radical, optionally substituted with one hydroxyl group; or an araliphatic or aromatic radical;
R₃ is a linear or branched alkylene chain, containing 1 to 6 carbon atoms, optionally substituted with one or more aryl groups; or an aralkylene chain, containing 7 to 10 carbon atoms;
n is O or 1; m is 1 or 2; n + m is 2;
or R₂ and R₃ form together with the nitrogen atom a 4-7 membered ring, optionally substituted with one or more carboxylic groups, and linked to oxygen with a covalent bond;
R₄ is a saccharidic portion bound to the previous molecule portion with a glucosidic linkage, said saccharidic portion being derived from a monosaccharide, a disaccharide or a trisaccharide, wherein the hydroxyl groups of the saccharides are optionally esterified with acetyl, sulphate or phosphate groups, and/or are substituted by one or more aminic groups, optionally N-acylated; for use as a medicament.

34. The saccharidic derivative according to claim 33, for treating acute and chronic diseases of the central nervous system associated with overstimulation of excitatory amino acids receptors.

35. The saccharidic derivative according to claim 34, wherein said diseases are selected from the group consisting of hypoxia-ischaemia, stroke. brain and spinal cord injuries, epilepsy, transient ischaemic attacks, neurolathyrism, amyotrophic lateral sclerosis, Huntington's Chorea. Alzheimer's disease, Parkinson's disease, primary dementia, dementia associated with viral pathologies and anoxy-ischaemic retinic diseases.

## Patentansprüche

1. Saccharid-Derivate von N-Acylalkylaminen der Formel (1): 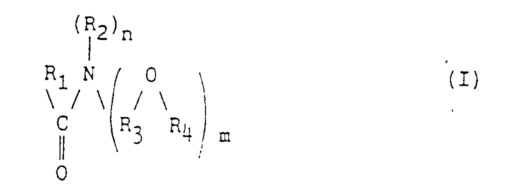 worin bedeuten:
R₁ den Rest einer gesättigten oder ungesättigten, linearen oder verzweigten aliphatischen Monocarbonsäure, die 1 bis 24 Kohlenstoffatome enthält und gegebenenfalls durch eine oder mehr Amin-, Alkohol-, Keton-Gruppen, heterocyclische, alicyclische oder polycyclische Gruppen substituiert ist; oder den Rest einer aromatischen oder heterocyclischen Monocarbonsäure, die 3 bis 24 Kohlenstoffatome enthält; oder den Rest einer gesättigten oder ungesättigten, aliphatischen, araliphatischen, aromatischen oder heterocyclischen Dicarbonsäure, die 2 bis 24 Kohlenstoffatome enthält und gegebenenfalls substituiert ist durch eine oder mehr Amin-, Alkohol- oder Keton-Gruppen;
R₂ H oder einen linearen oder verzweigten aliphatischen C₁-C₈-Rest, der gegebenenfalls substituiert ist durch eine Hydroxylgruppe; oder einen araliphatischen oder aromatischen Rest;
R₃ eine lineare oder verzweigte Alkylenkette, die 1 bis 6 Kohlenstoffatome enthält und gegebenenfalls substituiert ist durch eine oder mehr Arylgruppen; oder eine Aralkylenkette, die 7 bis 10 Kohlenstoffatome enthält;
n 0 oder 1;
m 1 oder 2;
n + m = 2;
oder worin R₂ und R₃ gemeinsam zusammen mit dem Stickstoffatom einen 4- bis 7-gliedrigen Ring bilden, der gegebenenfalls durch eine oder mehr Carboxylgruppen substituiert und mit einer kovalenten Bindung an Sauerstoff gebunden ist;
R₄ einen Saccharid-Abschnitt, der mit einer glucosidischen Bindung an den übrigen (vorhergehenden) Molekül-Abschnitt gebunden ist, wobei der genannte Saccharid-Abschnitt von einem Monosaccharid, einem Disaccharid oder einem Trisaccharid abgeleitet ist, wobei die Hydroxylgruppen der Saccharide gegebenenfalls mit Acetyl-, Sulfat- oder Phosphatgruppen verestert sind und/oder durch eine oder mehr Amingruppen, die gegebenenfalls N-acyliert sind, substituiert sind,
mit Ausnahme der folgenden Verbindungen:
2-O-(β-D-Glucopyranosyl)-N-decanoyl-aminoethanol;
2-O-(β-D-Glucopyranosyl)-N-dodecanoyl-aminoethanol;
2-O-(β-D-Glucopyranosyl)-N-myristoyl-aminoethanol;
2-O-(β-D-Glucopyranosyl)-N-palmitoyl-aminoethanol;
2-O-(β-D-Glucopyranosyl)-N-decanoyl-3-aminopropanol;
2-O-(β-D-Glucopyranosyl)-N-dodecanoyl-3-aminopropanol;
2-O-(β-D-Glucopyranosyl)-N-myristoyl-3-aminopropanol;
2-O-(β-D-Glucopyranosyl)-N-palmitoyl-5-aminopentanol;
2-O-(α-D-Glucopyranosyl)-N-decanoyl-aminoethanol;
2-O-(α-D-Glucopyranosyl)-N-decanoyl-3-aminopropanol.

2. Saccharid-Derivate nach Anspruch 1, worin die genannte Monocarbonsäure ausgewählt wird aus der Gruppe, die besteht aus Essigsäure, Capronsäure, Palmitinsäure, Ölsäure, Myristinsäure, Linolsäure, Stearinsäure, Laurinsäure, Nervonsäure, Arachidonsäure, ω-Hydroxypalmitinsäure, γ-Aminobuttersäure, y-Trimethyl-β-hydroxybutyrobetain und Derivaten davon, die an der Hydroxylgruppe acyliert sind, Benzoesäure, Trimethoxybenzoesäure, Nicotinsäure, Phenylanthranylsäure, Thioctsäure und Deoxycholsäure.

3. Saccharid-Derivate nach Anspruch 1, worin die genannte Dicarbonsäure ausgewählt wird aus der Gruppe, die besteht aus Fumarsäure, Azelainsäure, Oxalsäure, Bernsteinsäure, Glutarsäure, Pimelinsäure, Traumatinsäure, Maleinsäure und Malonsäure und Hydroxylhomologen davon, Phthalsäure, Folsäure und Chromoglycinsäure.

4. Saccharid-Derivate nach Anspruch 3, worin die zweite Carboxylgruppe der genannten Dicarbonsäure funktionalisiert ist unter Bildung eines Esters oder eines Amids.

5. Saccharid-Derivate nach Anspruch 3, worin die zweite Carboxylgruppe der genannten Dicarbonsäure an einen Molekülabschnitt gebunden ist, der identisch mit demjenigen ist, der an die erste Carboxylgruppe gebunden ist, unter Bildung eines symmetrischen Moleküls der Formel (II): 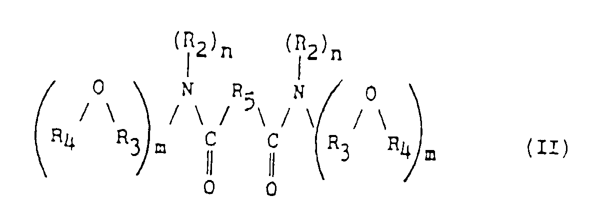 worin R₁, R₂, R₃, R₄, m und n wie in Anspruch 1 definiert sind und R₅ den divalenten Kohlenwasserstoff-Rest der genannten Dicarbonsäure bedeutet.

6. Saccharid-Derivate nach den Ansprüchen 1 bis 5, worin R₂ für H, n für die Zahl 1 und m für die Zahl 1 stehen.

7. Saccharid-Derivate nach den Ansprüchen 1 bis 5, worin R₂ ausgewählt wird aus der Gruppe, die besteht aus -CH₃, -C₂H₅, -CH₂OH und -C₂H₄OH; n für die Zahl 1 und m für die Zahl 1 stehen.

8. Saccharid-Derivate nach den Ansprüchen 1 bis 7, worin R₃ steht für Ethylen, Propylen, -(CH₂)₆-,

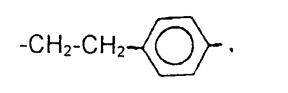

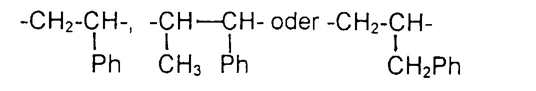

9. Saccharid-Derivate nach den Ansprüchen 1 bis 8, worin R₄ einen Monosaccharid-Abschnitt darstellt, der ausgewählt wird aus der Gruppe, die besteht aus D- und L-Ribose, D- und L-Glucose, D- und L-Galactose, D- und L-Mannose, D-Fructose, D- und L-Glucosamin, D-Galactosamin, D-Mannosamin, Glucuronsäure und Sialinsäure.

10. Saccharid-Derivate nach den Ansprüchen 1 bis 8, worin R₄ steht für einen Disaccharid-Abschnitt, der ausgewählt wird aus der Gruppe, die besteht aus Lactose, Maltose, Sialyl-Glucose und Sialyl-Galactose.

11. Saccharid-Derivate nach den Ansprüchen 1 bis 8, worin R₄ steht für den Trisaccharid-Abschnitt von Sialyllactose.

12. 2-O-(β-D-Glucopyranosyl)-N-acetyl-aminoethanol.

13. 2-O-(β-D-Galactopyranosyl)-N-palmitoyl-aminoethanol.

14. 2-O-(β-D-Galactopyranosyl)-N-benzoyl-aminoethanol.

15. 2-O-(β-D-Glucopyranosyl)-N-myristoleoyl-aminoethanol.

16. 2-O-(β-D-Glucopyranosyl)-N-oleoyl-aminopropanol.

17. 2-O-(β-D-Glucopyranosyl)-N-stearoyl-aminoethanol.

18. Verfahren zur Herstellung der Saccharid-Derivate nach Anspruch 1, bei dem man ein Amid der Formel: 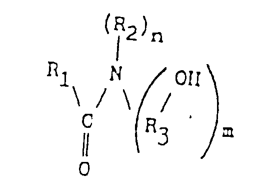 worin R₁, R₂, R₃, n und m wie in Anspruch 1 definiert sind, mit einem reaktionsfähigen Saccharid-Derivat in einem wasserfreien und aprotischen Lösungsmittel in Gegenwart von equimolaren Mengen eines Reaktionspromotors bei niedriger Temperatur reagieren läßt.

19. Verfahren nach Anspruch 18, bei dem das genannte Lösungsmittel Acetonitril oder Dichlormethan oder eine Mischung davon ist.

20. Verfahren nach Anspruch 18, bei dem der genannte Reaktionspromotor ein Silbersalz ist.

21. Verfahren nach Anspruch 20, bei dem das genannte Silbersalz Silbertrifluoromethansulfonat ist.

22. Verfahren zur Herstellung der Saccharid-Derivate nach Anspruch 1, das die folgenden Stufen umfaßt:
A) Umsetzung eines Aminoalkohols der Formel R₂-NH-R₃-OH, worin R₂ und R₃ wie in Anspruch 1 definiert sind, mit einem Saccharid in Gegenwart einer starken Säure unter einer Stickstoff-Atmosphäre; und
B) N-Acylierung des in der Stufe (A) erhaltenen Reaktionsprodukts mit einem aktivierten Derivat von R₁-COOH, worin R₁ wie in Anspruch 1 definiert ist.

23. Verfahren nach Anspruch 22, bei dem die Stufe (A) in einem inerten Lösungsmittel durchgeführt wird.

24. Verfahren nach Anspruch 23, bei dem in der Stufe (A) die genannte starke Säure Methansulfonsäure ist.

25. Pharmazeutische Zusammensetzungen, die als Wirkstoff (aktives Prinzip) mindestens ein Saccharid-Derivat von N-Acylalkylaminen der Formel (I): 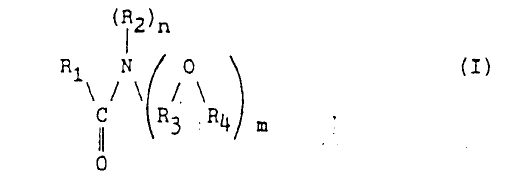 worin bedeuten:
R₁ den Rest einer gesättigten oder ungesättigten, linearen oder verzweigten aliphatischen Monocarbonsäure, die 1 bis 24 Kohlenstoffatome enthält und in der aliphatischen Kette gegebenenfalls durch eine oder mehr Amin-, Alkohol-, Keton-Gruppen, heterocyclische, alicyclische oder polycyclische Gruppen substituiert ist; oder den Rest einer aromatischen oder heterocyclischen Monocarbonsäure, die 3 bis 24 Kohlenstoffatome enthält; oder den Rest einer gesättigten oder ungesättigten, aliphatischen, araliphatischen, aromatischen oder heterocyclischen Dicarbonsäure, die 2 bis 24 Kohlenstoffatome enthält und gegebenenfalls substituiert ist durch eine oder mehr Amin-, Alkohol- oder Keton-Gruppen;
R₂ H oder einen linearen oder verzweigten aliphatischen C₁-C₈-Rest, der gegebenenfalls substituiert ist durch eine Hydroxylgruppe; oder einen araliphatischen oder aromatischen Rest;
R₃ eine lineare oder verzweigte Alkylenkette, die 1 bis 6 Kohlenstoffatome enthält und gegebenenfalls substituiert ist durch eine oder mehr Arylgruppen; oder eine Aralkylenkette, die 7 bis 10 Kohlenstoffatome enthält;
n 0 oder 1;
m 1 oder 2;
n + m = 2;
oder worin R₂ und R₃ gemeinsam zusammen mit dem Stickstoffatom einen 4- bis 7-gliedrigen Ring bilden, der gegebenenfalls durch eine oder mehr Carboxylgruppen substituiert und mit einer covalenten Bindung an Sauerstoff gebunden ist;
R₄ einen Saccharid-Abschnitt, der mit einer glucosidischen Bindung an den übrigen (vorhergehenden) Molekül-Abschnitt gebunden ist, wobei der genannte Saccharid-Abschnitt von einem Monosaccharid, einem Disaccharid oder einem Trisaccharid abgeleitet ist, wobei die Hydroxylgruppen der Saccharide gegebenenfalls mit Acetyl-, Sulfat- oder Phosphatgruppen verestert sind und/oder durch eine oder mehr Amingruppen, die gegebenenfalls N-acyliert sind, substituiert sind,
in Kombination mit geeigneten Exzipienten und/oder Verdünnungsmitteln enthalten.

26. Pharmazeutische Zusammensetzungen nach Anspruch 25, die auf oralem oder parenteralem Wege verabreichbar sind.

27. Pharmazeutische Zusammensetzungen nach Anspruch 26, die in Form von Granulat-Pulvern, Tabletten, Pillen oder Kapseln oral verabreichbar sind.

28. Pharmazeutische Zusammensetzungen nach Anspruch 26, die intravenös, subkutan oder intramuskulär verabreichbar sind.

29. Pharmazeutische Zusammensetzungen nach Anspruch 25, die auf topischem oder transdermalem Wege verabreichbar sind.

30. Pharmazeutische Zusammensetzungen nach Anspruch 25, die für die ophthalmische Verwendung in Form von Augenwässern oder Salben geeignet sind.

31. Pharmazeutische Zusammensetzungen nach Anspruch 25; mit denen der Wirkstoff (das aktive Prinzip) in einer Dosis in dem Bereich von 0,1 bis 100 mg/kg/Tag mindestens 30 Tage lang verabreicht wird.

32. Pharmazeutische Zusammensetzungen nach Anspruch 31, bei denen die genannte Dosis in dem Bereich von 1 bis 30 mg/kg/Tag liegt.

33. Saccharid-Derivat von N-Acylalkylaminen der Formel (I): 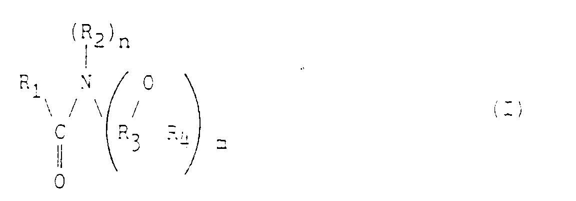 worin bedeuten:
R₁ den Rest einer gesättigten oder ungesättigten, linearen oder verzweigten aliphatischen Monocarbonsäure, die 1 bis 24 Kohlenstoffatome enthält und gegebenenfalls in der aliphatischen Kette durch eine oder mehr Amin-, Alkohol-, Keton-Gruppen, heterocyclische, alicyclische oder polycyclische Gruppen substituiert ist; oder den Rest einer aromatischen oder heterocyclischen Monocarbonsäure, die 3 bis 24 Kohlenstoffatome enthält; oder den Rest einer gesättigten oder ungesättigten, aliphatischen, araliphatischen, aromatischen oder heterocyclischen Dicarbonsäure, die 2 bis 24 Kohlenstoffatome enthält und gegebenenfalls substituiert ist durch eine oder mehr Amin-, Alkohol- oder Keton-Gruppen;
R₂ H oder einen linearen oder verzweigten aliphatischen C₁-C₈-Rest, der gegebenenfalls substituiert ist durch eine Hydroxylgruppe; oder einen araliphatischen oder aromatischen Rest;
R₃ eine lineare oder verzweigte Alkylenkette, die 1 bis 6 Kohlenstoffatome enthält und gegebenenfalls substituiert ist durch eine oder mehr Arylgruppen; oder eine Aralkylenkette, die 7 bis 10 Kohlenstoffatome enthält;
n 0 oder 1;
m 1 oder 2;
n + m = 2;
oder worin R₂ und R₃ gemeinsam zusammen mit dem Stickstoffatom einen 4- bis 7-gliedrigen Ring bilden, der gegebenenfalls durch eine oder mehr Carboxylgruppen substituiert und mit einer covalenten Bindung an Sauerstoff gebunden ist;
R₄ einen Saccharid-Abschnitt, der mit einer glucosidischen Bindung an den übrigen (vorhergehenden) Molekül-Abschnitt gebunden ist, wobei der genannte Saccharid-Abschnitt von einem Monosaccharid, einem Disaccharid oder einem Trisaccharid abgeleitet ist, wobei die Hydroxylgruppen der Saccharide gegebenenfalls mit Acetyl-, Sulfat- oder Phosphatgruppen verestert sind und/oder durch eine oder mehr Amingruppen, die gegebenenfalls N-acyliert sind, substituiert sind,
für die Verwendung als Arzneimittel.

34. Saccharid-Derivat nach Anspruch 33 für die Behandlung akuter und chronischer Erkrankungen des Zentralnervensystems, die mit der Überstimulierung von exzitatorischen Aminosäure-Rezeptoren im Zusammenhang stehen.

35. Saccharid-Derivat nach Anspruch 34, wobei die genannten Erkrankungen ausgewählt werden aus der Gruppe, die besteht aus Hypoxie-lschämie, Schlaganfall, Gehirn- und Rückenmarks-Verletzungen, Epilepsie, vorübergehenden ischämischen Anfällen, Neurolathyrismus, amyotropher Lateralsklerose, Chorea-Huntington, Alzheimer-Erkrankung, Parkinson-Erkrankung, primärer Demenz, Demenz, die mit viralen Pathologien im Zusammenhang steht, und anoxie-ischämischen Retina-Erkrankungen.

## Revendications

1. Dérivés saccharides de N-acyl alkylamines de formule (I): 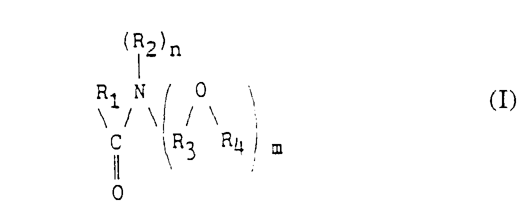 dans laquelle :
R₁ est le radical d'un acide monocarboxylique aliphatique saturé ou insaturé, à chaîne linéaire ou ramifiée, contenant 1 à 24 atomes de carbone, substitué éventuellement avec un ou plusieurs groupes amino, alcool, cétone, hétérocycliques, alicycliques ou polycycliques; ou est le radical d'un acide monocarboxylique aromatique ou hétérocyclique contenant 3 à 24 atomes de carbone;
ou le radical d'un acide dicarboxylique aliphatique, araliphatique, aromatique ou hétérocyclique saturé ou insaturé, contenant 2 à 24 atomes de carbone, éventuellement substitué avec un ou plusieurs groupes amino, alcool ou cétone;
R₂ est un atome d'hydrogène ou un radical aliphatique en C₁₋₈ à chaîne linéaire ou ramifiée éventuellement substitué avec un groupe hydroxy; ou un radical araliphatique ou aromatique;
R₃ est une chaîne alkylène linéaire ou ramifiée contenant 1 à 6 atomes de carbone, éventuellement substitué avec un ou plusieurs groupes aryles; ou une chaîne aralkylène contenant 7 à 10 atomes de carbone;
n est 0 ou 1; m est 1 ou 2; n + m est 2;
ou bien R₂ et R₃ forment ensemble avec l'atome d'azote un noyau de 4 à 7 chaînons, éventuellement substitué avec un ou plusieurs groupes carboxyliques, et lié à un atome d'oxygène par une liaison covalente;
R₄ est une partie saccharide liée à la partie de la molécule précédente avec une liaison glucosidique, cette partie saccharide provenant d'un monosaccharide, d'un disaccharide ou d'un trisaccharide, où les groupes hydroxy des saccharides sont éventuellement estérifiés avec des groupes acétyle, sulfate ou phosphate, et/ou sont substitués par un ou plusieurs groupes amino, éventuellement N-acylés,
à l'exclusion des composés suivants :
- 2-O-(β-D-glucopyranosyl)-N-décanoyl-aminoéthanol;
- 2-O-(β-D-glucopyranosyl)-N-dodécanoyl-aminoéthanol;
- 2-O-(β-D-glucopyranosyl)-N-myristoyl-aminoéthanol;
- 2-O-(β-D-glucopyranosyl)-N-palmitoyl-aminoéthanol;
- 2-0-(β-D-glucopyranosyl)-N-décanoyl-3-aminopropanol;
- 2-0-(β-D-glucopyranosyl)-N-dodécanoyl-3-aminopropanol;
- 2-0-(β-D-glucopyranosyl)-N-myristoyl-3-aminopropanol;
- 2-O-(β-D-glucopyranosyl)-N-palmitoyl-5-aminopentanol;
- 2-O-(α-D-glucopyranosyl)-N-décanoyl-aminoéthanol;
- 2-O-(α-D-glucopyranosyl)-N-décanoyl-3-aminopropanol.

2. Dérivés saccharides suivant la revendication 1, dans lesquels cet acide monocarboxylique est choisi dans le groupe consistant en acides acétique, caproïque, palmitique, oléique, myristique, linoléique, stéarique, laurique, nervonique, arachidonique, ω-hydroxy-palmitique, γ-aminobutyrique, γ-triméthyl-β-hydroxybutyrobétaine et dérivés de ceux-ci acylés au niveau du groupe hydroxy, acides benzoïque, triméthoxybenzoïque, nicotinique, phénylanthranylique, thioctique et désoxycholique.

3. Dérivés saccharides suivant la revendication 1, dans lesquels cet acide dicarboxylique est choisi dans le groupe consistant en acides fumarique, azélaïque, oxalique, succinique, glutarique, pimélique, traumatique, maléique et malonique et homologues hydroxy de ceux-ci, acides phtalique, folique et chromoglycique.

4. Dérivés saccharides suivant la revendication 3, dans lesquels le second groupe carboxy de cet acide dicarboxylique est fonctionnalisé pour donner un ester ou un amide.

5. Dérivés saccharides suivant la revendication 3, dans lesquels le second groupe carboxy de cet acide dicarboxylique est lié à une partie de molécule identique à celle qui est liée au premier groupe carboxy, avec formation d'une molécule symétrique de formule (II) : 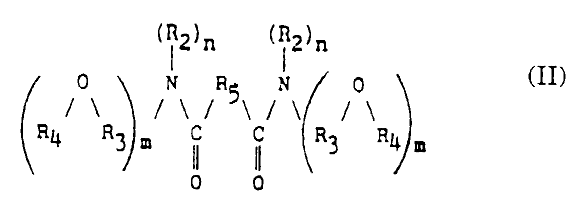 dans laquelle R₁, R₂, R₃, R₄, m et n sont tels que définis dans la revendication 1, et R₅ est le radical divalent hydrocarboné de cet acide dicarboxylique.

6. Dérivés saccharides suivant les revendications 1 à 5, dans lesquels R₂ est un atome d'hydrogène, n est 1 et m est 1.

7. Dérivés saccharides suivant les revendications 1 à 5, dans lesquels R₂ est choisi dans le groupe consistant en -CH₃, -C₂H₅, -CH₂OH et -C₂H₄OH; n est 1 et m est 1.

8. Dérivés saccharides suivant les revendications 1 à 7, dans lesquels R₃ est un groupe éthylène, propylène, -(CH₂)₆-,

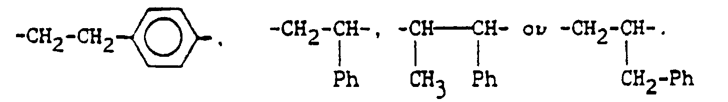

9. Dérivés saccharides suivant les revendications 1 à 8, dans lesquels R₄ est une partie monosaccharide choisie dans le groupe consistant en D- et L-ribose, D- et L-glucose, D- et L-galactose, D- et L-mannose, D-fructose, D- et L-glucosamine, D-galactosamine, D-mannosamine, acide glucuronique et acide sialique.

10. Dérivés saccharides suivant les revendications 1 à 8, dans lesquels R₄ est une partie disaccharide choisie dans le groupe consistant en lactose, maltose, sialyl-glucose et sialyl-galactose.

11. Dérivés saccharides suivant les revendications 1 à 8, dans lesquels R₄ est la partie trisaccharide du sialyl-lactose.

12. 2-O-(β-D-glucopyranosyl)-N-acétyl-aminoéthanol.

13. 2-O-(β-D-galactopyranosyl)-N-palmitoyl-aminoéthanol.

14. 2-O-(β-D-galactopyranosyl)-N-benzoyl-aminoéthanol.

15. 2-O-(β-D-glucopyranosyl)-N-myristoloyl-aminoéthanol.

16. 2-O-(β-D-glucopyranosyl)-N-oléoyl-aminoéthanol.

17. 2-O-(β-D-glucopyranosyl)-N-stéaroyl-aminoéthanol.

18. Procédé pour la préparation des dérivés saccharides suivant la revendication 1, dans lequel un amide de formule : 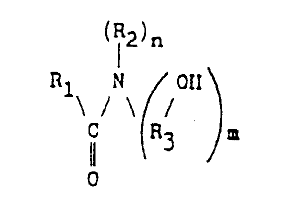 dans laquelle R₁, R₂, R₃, m et n sont tels que définis dans la revendication 1, est traité avec un dérivé saccharide réactif, dans un solvant anhydre et aprotique, en présence de quantités équimolaires d'un activateur de réaction, à basse température.

19. Procédé suivant la revendication 18, dans lequel ce solvant est l'acétonitrile ou le dichlorométhane ou des mélanges de ceux-ci.

20. Procédé suivant la revendication 18, dans lequel cet activateur de réaction est un sel d'argent.

21. Procédé suivant la revendication 20, dans lequel ce sel d'argent est le trifluorométhane sulfonate d'argent.

22. Procédé pour la préparation des dérivés saccharides suivant la revendication 1, comprenant les étapes suivantes :
(A) réaction d'un aminoalcool de formule R₂-NH-R₃-OH, dans laquelle R₂ et R₃ sont tels que définis dans la revendication 1, avec un saccharide, en présence d'un acide fort, dans une atmosphère d'azote;
(B) N-acylation du produit réactionnel obtenu dans l'étape (A) avec un dérivé activé de R₁-COOH, dans lequel R₁ est tel que défini dans la revendication 1.

23. Procédé suivant la revendication 22, dans lequel l'étape (A) est conduite dans un solvant inerte.

24. Procédé suivant la revendication 23, dans lequel dans l'étape (A), cet acide fort est l'acide méthanesulfonique.

25. Compositions pharmaceutiques comprenant en tant que principe actif au moins un dérivé saccharide de N-acyl alkylamines de formule (I): 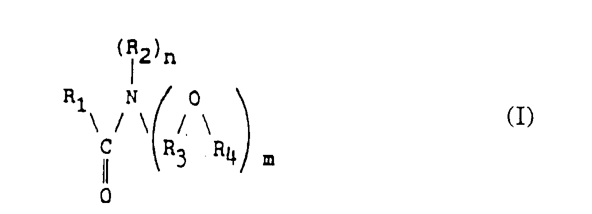 dans laquelle :
R₁ est le radical d'un acide monocarboxylique aliphatique saturé ou insaturé, à chaîne linéaire ou ramifiée, contenant 1 à 24 atomes de carbone, dont la chaîne aliphatique est substituée éventuellement avec un ou plusieurs groupes amino, alcool, cétone, hétérocycliques, alicycliques ou polycycliques;
ou est le radical d'un acide monocarboxylique aromatique ou hétérocyclique contenant 3 à 24 atomes de carbone;
ou le radical d'un acide dicarboxylique aliphatique, araliphatique, aromatique ou hétérocyclique saturé ou insaturé, contenant 2 à 24 atomes de carbone, éventuellement substitué avec un ou plusieurs groupes amino, alcool ou cétone;
R₂ est un atome d'hydrogène ou un radical aliphatique en C₁₋₈ à chaîne linéaire ou ramifiée éventuellement substitué avec un groupe hydroxy; ou un radical araliphatique ou aromatique;
R₃ est une chaîne alkylène linéaire ou ramifiée contenant 1 à 6 atomes de carbone, éventuellement substitué avec un ou plusieurs groupes aryles; ou une chaîne aralkylène contenant 7 à 10 atomes de carbone;
n est 0 ou 1; m est 1 ou 2; n + m est 2;
ou bien R₂ et R₃ forment ensemble avec l'atome d'azote un noyau de 4 à 7 chaînons, éventuellement substitué avec un ou plusieurs groupes carboxyliques, et lié à un atome d'oxygène par une liaison covalente;
R₄ est une partie saccharide liée à la partie de la molécule précédente avec une liaison glucosidique, cette partie saccharide provenant d'un monosaccharide, d'un disaccharide ou d'un trisaccharide, où les groupes hydroxy des saccharides sont éventuellement estérifiés avec des groupes acétyle, sulfate ou phosphate, et/ou sont substitués par un ou plusieurs groupes amino, éventuellement N-acylés,
en combinaison avec des excipients et/ou diluants convenables.

26. Compositions pharmaceutiques suivant la revendication 25, pouvant être administrées par voie orale ou parentérale.

27. Compositions pharmaceutiques suivant la revendication 26, pouvant être administrées par voie orale sous la forme de poudres granulaires, comprimés, pilules ou gélules.

28. Compositions pharmaceutiques suivant la revendication 26, pouvant être administrées par voie intraveineuse, sous-cutanée ou intramusculaire.

29. Compositions pharmaceutiques suivant la revendication 25, pouvant être administrées par voie topique ou transdermique.

30. Compositions pharmaceutiques suivant la revendication 25, convenables pour un usage ophtalmique sous la forme de collyres ou de pommades.

31. Compositions pharmaceutiques suivant la revendication 25, dans lesquelles ce principe actif est administré à une dose comprise dans la gamme de 0,1 à 100 mg/kg par jour, pendant au moins 30 jours.

32. Compositions pharmaceutiques suivant la revendication 31, dans lesquelles cette dose est comprise dans la gamme de 1 à 30 mg/kg par jour.

33. Dérivé saccharide de N-acyl alkylamines de formule (I): 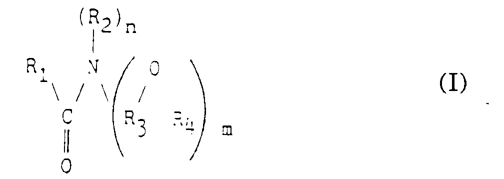 dans laquelle :
R₁ est le radical d'un acide monocarboxylique aliphatique saturé ou insaturé, à chaîne linéaire ou ramifiée, contenant 1 à 24 atomes de carbone, dont la chaîne aliphatique est substituée éventuellement avec un ou plusieurs groupes amino, alcool, cétone, hétérocycliques, alicycliques ou polycycliques;
ou est le radical d'un acide monocarboxylique aromatique ou hétérocyclique contenant 3 à 24 atomes de carbone;
ou le radical d'un acide dicarboxylique aliphatique, araliphatique, aromatique ou hétérocyclique saturé ou insaturé, contenant 2 à 24 atomes de carbone, éventuellement substitué avec un ou plusieurs groupes amino, alcool ou cétone;
R₂ est un atome d'hydrogène ou un radical aliphatique en C₁₋₈ à chaîne linéaire ou ramifiée, éventuellement substitué avec un groupe hydroxy; ou un radical araliphatique ou aromatique;
R₃ est une chaîne alkylène linéaire ou ramifiée contenant 1 à 6 atomes de carbone, éventuellement substituée avec un ou plusieurs groupes aryles; ou une chaîne aralkylène contenant 7 à 10 atomes de carbone;
n est 0 ou 1; m est 1 ou 2; n + m est 2;
ou bien R₂ et R₃ forment ensemble avec l'atome d'azote un noyau de 4 à 7 chaînons, éventuellement substitué avec un ou plusieurs groupes carboxyliques, et lié à un atome d'oxygène par une liaison covalente;
R₄ est une partie saccharide liée à la partie de la molécule précédente avec une liaison glucosidique, cette partie saccharide provenant d'un monosaccharide, d'un disaccharide ou d'un trisaccharide, où les groupes hydroxy des saccharides sont éventuellement estérifiés avec des groupes acétyle, sulfate ou phosphate, et/ou sont substitués par un ou plusieurs groupes amino, éventuellement N-acylés,
utilisable en tant que médicament.

34. Dérivé saccharide suivant la revendication 33, pour le traitement d'affections aiguës ou chroniques du système nerveux central associées à une stimulation excessive des récepteurs d'acides aminés excitatoires.

35. Dérivé saccharide suivant la revendication 34, où ces affections sont choisies dans le groupe consistant en hypoxie-ischémie, accident vasculaire cérébral constitué, lésions cérébrales et de la moëlle épinière, épilepsie, accidents ischémiques transitoires, neurolathyrisme, sclérose latérale amyotrophique, chorée de Huntington, maladie d'Alzheimer, maladie de Parkinson, démence primaire, démence associée à des pathologies virales et affections rétiniques anoxy-ischémiques.
